(19) Europäisches Patentamt
European Patent Office
Office européen des brevets

(11) EP 3 903 676 A1

(12) **EUROPEAN PATENT APPLICATION**

(43) Date of publication:
**03.11.2021 Bulletin 2021/44**

(51) Int Cl.:
***A61B 5/026*** *(2006.01)* ***A61B 5/00*** *(2006.01)*

(21) Application number: **21171362.3**

(22) Date of filing: **29.04.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2020 US 202063018301 P**
**26.04.2021 US 202117240267**

(71) Applicant: **Facebook Technologies, LLC**
**Menlo Park, CA 94025 (US)**

(72) Inventors:
- **Sie, Edbert Jarvis**
  **Menlo Park, 94025 (US)**
- **Marsili, Francesco**
  **Menlo Park, 94025 (US)**

(74) Representative: **Murgitroyd & Company**
**Murgitroyd House**
**165-169 Scotland Street**
**Glasgow G5 8PL (GB)**

(54) **MULTI-SPECKLE DIFFUSE CORRELATION SPECTROSCOPY**

(57) In some examples, an apparatus may include a laser, a source fiber that delivers the laser radiation to an object, and a detector fiber that receives scattered laser radiation and illuminates a detector array with the scattered laser radiation to form speckles on the detector array. In some examples, the object may be directed illuminated by a laser. The detector array may include a plurality of detectors, and may be positioned to receive the scattered laser radiation from the end of the detector fiber. The distance between the detector array and the end of the detector fiber may be adjustable. A controller may be configured to receive detector data from the detector array, determine a time-dependent intensity autocorrelation function for each detector of a plurality of detectors, and determine an ensemble average autocorrelation function. The apparatus may provide information relating to dynamic processes within the object. Various other methods, systems, and computer-readable media are also disclosed.

200
SMF
204
206
208
ρ
216
214
MMF
218
222
z
CW Laser
785 nm
Object
32 x 32
SPAD Array
202
210
212
220
Controller
230

FIG. 2A

EP 3 903 676 A1

## Description

## SUMMARY OF THE INVENTION

According to the present invention there is provided an apparatus comprising: a laser, configured to provide laser radiation; a detector fiber, having a collector end configured to receive scattered laser radiation and a detector end; a detector array comprising a plurality of detectors positioned to receive the scattered laser radiation from the detector end of the detector fiber; and a controller, configured to: receive detector data for each detector of the plurality of detectors; determine a time-dependent intensity autocorrelation function for each detector of the plurality of detectors; and determine an ensemble average autocorrelation function based on the time-dependent intensity autocorrelation function for each detector of the plurality of detectors.

Optionally, the apparatus further comprises a distance adjuster configured to adjust a distance between the detector end of the detector fiber and the detector array.

Optionally the apparatus further comprises at least one optical element located between the detector end of the detector fiber and the detector array.

Optionally, the plurality of detectors includes an arrangement of single-photon avalanche diodes.

Optionally the plurality of detectors comprises at least 1000 detectors.

Optionally the apparatus further comprises a source fiber, wherein: the source fiber has a source end configured to receive the laser radiation from the laser and a delivery end; and the source fiber includes a single-mode fiber.

Optionally the detector fiber includes a multimode fiber.

The apparatus may optionally be configured so that the scattered laser radiation emerges from the detector end of the detector fiber to form a plurality of speckles on the detector array.

Optionally the laser radiation has a wavelength of between 700 nm and 1200 nm.

Optionally, the laser radiation has a coherence length of at least 1 m.

The apparatus may further comprise a beam-splitter configured to direct unscattered laser radiation to the detector array.

The controller may optionally be configured to provide a controller output including time determination based on the ensemble average autocorrelation function.

Optionally, the time determination is related to fluid flow dynamics within an object illuminated by the laser radiation.

The apparatus may optionally be a wearable apparatus configured to be worn by a user.

The apparatus may optionally be configured so that the laser radiation is directed into a body part of the user when the apparatus is worn by the user, and the collector end of the detector fiber may optionally receive the scattered laser radiation from the body part of the user.

Optionally the apparatus is a head-mounted device and the body part is a head of the user.

Optionally the apparatus includes at least one band configured to attach the apparatus to the body part of the user.

According to the present invention there is further provided a method comprising: collecting scattered laser radiation using a detector fiber; illuminating a detector array using the scattered laser radiation to form a plurality of speckles on the detector array; and determining an ensemble average correlation function based on time-dependent intensity correlation functions for each of a plurality of detectors of the detector array.

Optionally the method further comprises adjusting a distance or a lens position between an end of the detector fiber and the detector array so that a speckle size on the detector array is approximately equal to a detector area within the plurality of detectors.

Optionally the scattered laser radiation has a wavelength of between 700 nm and 1200 nm.

Optionally the method further comprises illuminating an object using laser radiation, and optionally determining a characteristic time related to fluid flow within the object from the ensemble average correlation function.

## BRIEF DESCRIPTION OF THE DRAWINGS

The accompanying drawings illustrate a number of examples of the invention and are a part of the specification. Together with the following description, these drawings demonstrate and explain various principles of the present disclosure.

FIG. 1 is a diagram of the use of an example device for speckle correlation spectroscopy.
FIGS. 2A - 2E further illustrate an example apparatus for speckle correlation spectroscopy and its operation.
FIG. 3 is a block diagram of an example system for fiber-based speckle contrast optical spectroscopy.
FIG. 4 shows an example optical fiber projecting a speckle field onto a detector array.

FIGS. 5A - 5D show example improvements in the signal to noise ratio through formation of an ensemble average autocorrelation function.

FIGS. 6A and 6B show SNR improvements as a function of integration time and count rate per pixel, respectively.

FIG. 7A - 7C further illustrates speckle formation on a detector array.

FIGS. 8A - 8H illustrate speckle formation on a detector array and effects of adjusting the distance between the detector end of the fiber and the detector array.

FIGS. 9A - 9F illustrate cluster sizes for projections of speckles onto a detector array.

FIGS. 10A - 10C show that the cluster length may remain equal the speckle diameter even as the speckle diameter is increased.

FIGS. 11A - 11F illustrate cluster and ensemble averaging in formation of an autocorrelation function, and associated noise characteristics.

FIGS. 12A and 12B illustrate the effect of fiber core diameter on SNR.

FIGS. 13A - 13E show the effects of hot pixels in a detector array on the intensity autocorrelation function ($g_2$) and SNR.

FIGS. 14A - 14C further illustrate characteristics of hot pixels in a detector array.

FIGS. 15A and 15B show example fitting functions for an intensity autocorrelation function.

FIGS. 16A - 16C illustrate the use of pulsed laser radiation to increase the intensity of laser pulses while maintaining the same average laser power.

FIGS. 17A - 17B show the effects of speckle tracking using object detection.

FIGS. 18A - 18J show further example characteristics of an ensemble average autocorrelation function.

FIG. 19 is a flow diagram of an example method for fiber-based speckle contrast optical spectroscopy.

FIG. 20 is a flow diagram of a further example method for fiber-based speckle contrast optical spectroscopy.

FIG. 21 is an illustration of exemplary augmented-reality glasses that may be used in connection with embodiments of this disclosure.

FIG. 22 is an illustration of an exemplary virtual-reality headset that may be used in connection with embodiments of this disclosure.

Throughout the drawings, identical reference characters and descriptions indicate similar, but not necessarily identical, elements.

## DETAILED DESCRIPTION OF EXAMPLES OF THE INVENTION

The present disclosure is generally directed to object characterization using light scattering. As is explained in greater detail below, the invention may include an apparatus that includes a laser, a source fiber that delivers the laser radiation to an object, and a detector fiber that receives scattered laser radiation and illuminates a detector array with the scattered laser radiation to form speckles on the detector array. The detector array may include a plurality of detectors, and may be positioned to receive the scattered laser radiation from the end of the detector fiber. The distance between the detector array and the end of the detector fiber may be adjustable, and may be coupled optically by additional optical elements such as lenses, polarizers, filters, splitters, and combiners. A controller may be configured to receive detector data from the detector array, determine a time-dependent intensity autocorrelation function for each detector of a plurality of detectors, and determine an ensemble average autocorrelation function. The apparatus may provide information relating to dynamic processes within the object, such as fluid flow. In some examples, blood flow within a body part of a user may be characterized using multi-speckle diffuse correlation spectroscopy (mDCS).

Blood flow in specific areas of the brain may correlate with neuronal activity in those areas, indicating specific brain functions (such as the use of particular words, an emotion, an intent to select or interact with an object within a real or virtual environment, a desire to select an option such as a menu option, a desire to control a real or virtual device, a desire to operate a computer interface device such as a mouse, a desire to enter one or more alphanumeric characters, or other brain function). Thus, observing blood flow may provide the basis for a brain-computer interface. Light directed at a point of a person's head may penetrate and diffuse through that area of the head, creating a speckle field. Changes in the speckle field over time may provide information about blood flow in the targeted area, providing a non-invasive method for a brain-computer interface. An imaging array fed by a corresponding array of multi-mode fibers can penetrate hair and thus collect light to observe the speckle field with minimal interference from a user's hair. In addition, correlating speckles in the speckle field to pixels in the imaging array on a N:1 speckle-to-pixel basis may provide a high signal-to-noise ratio. In some approaches, an apparatus may be configured to obtain an approximately 1:1 speckle-to-pixel ratio, and then the speckle diameter may be adjusted to at least approximately optimize the signal-to-noise ratio (SNR). This may lead to an N:1 speckle-to-pixel ratio, where N may be greater than 1 (e.g., approximately 2, 3, or 4, or within the range 1.5 - 5, such as between approximately 2 and approximately 4), depending on background noise. An example apparatus may be operated with a 1:1 speckle-to-pixel ratio at the detector array, or may operate with a N:1 speckle-to-pixel ratio at the detector array, where $N \geq 1$ (e.g., where N is between approximately 2 and approximately 4). In some examples, identification of a brain function may be correlated with an eye-tracking system to identify an object or virtual representation that the person desires to interact with.

In some examples, the imaging array may be large (e.g., 32x32, or N x M where N and M are both integers and N x M > 1000). In some examples, an apparatus may include a 128 x 128 detector array (e.g., a SPAD array), such as a 512 x 512 detector array (e.g., a SPAD array).

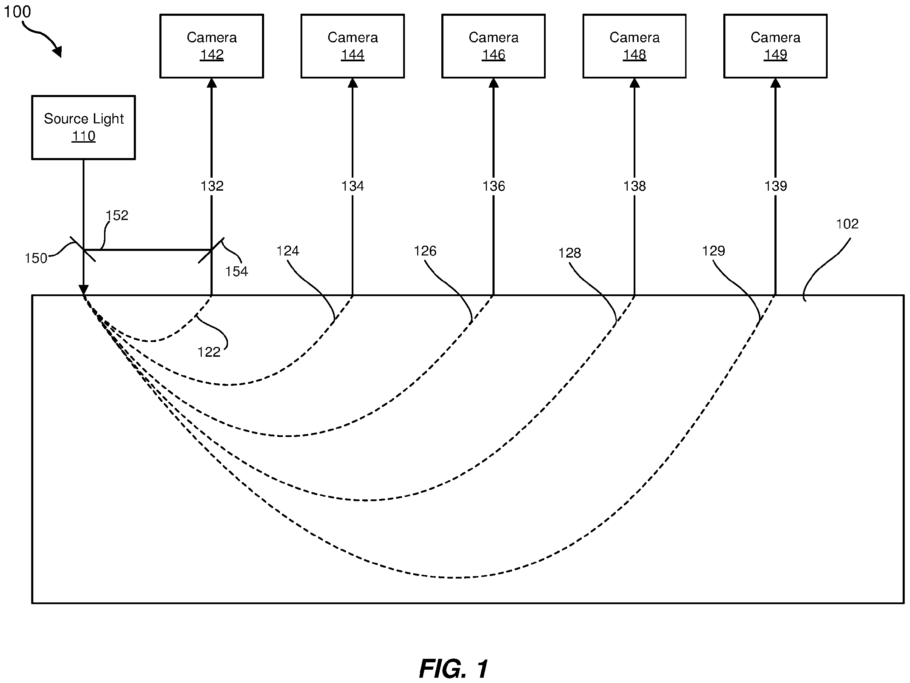

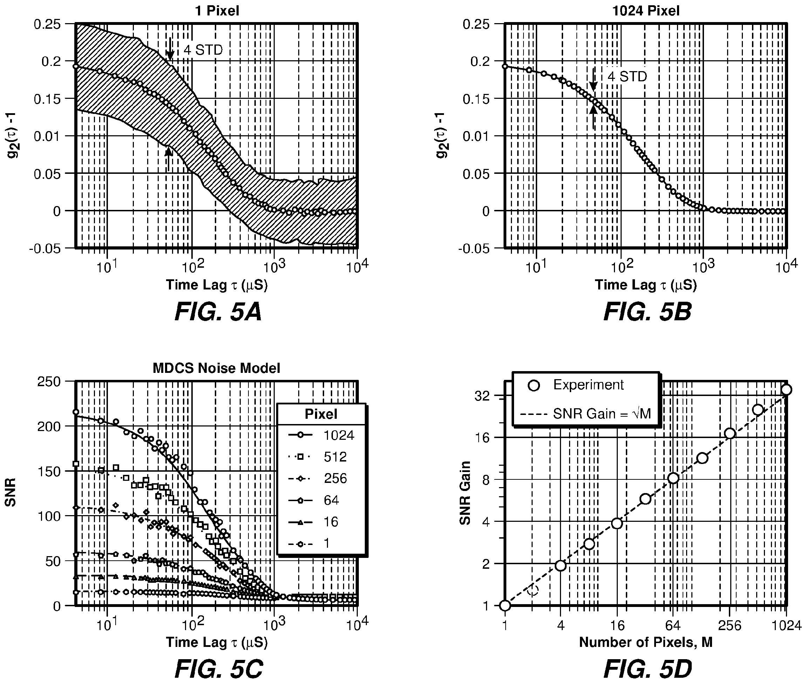

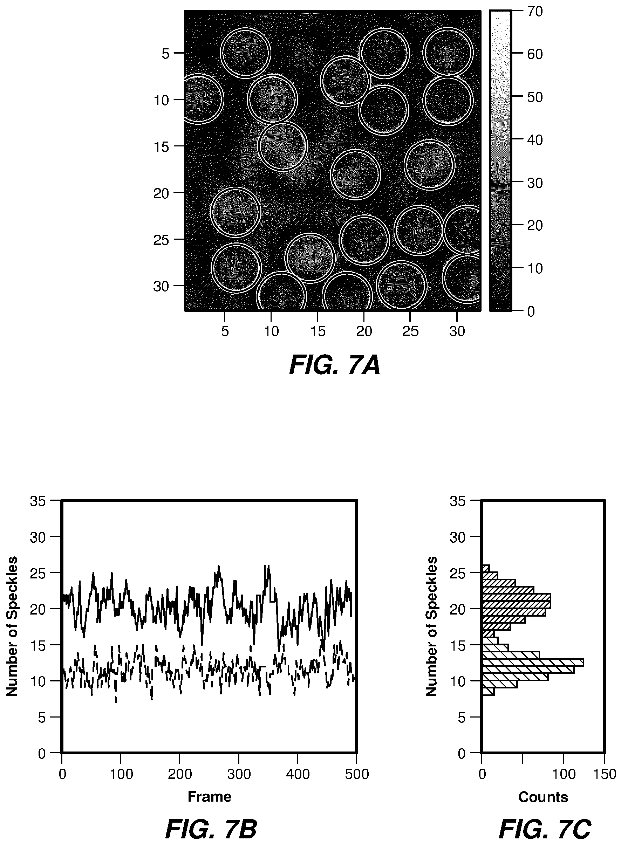

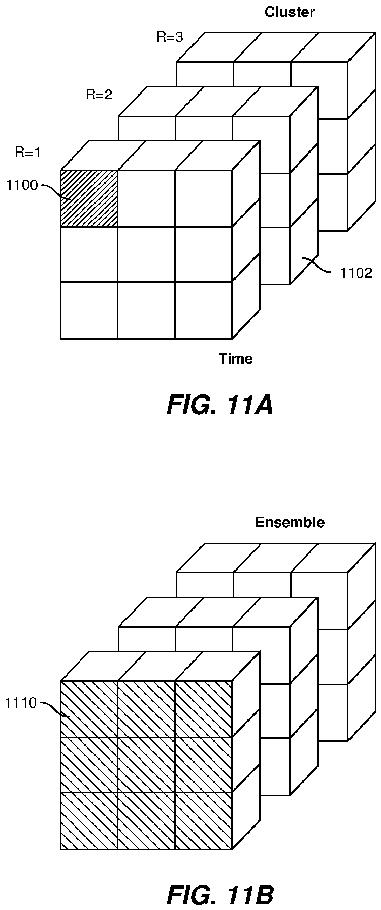

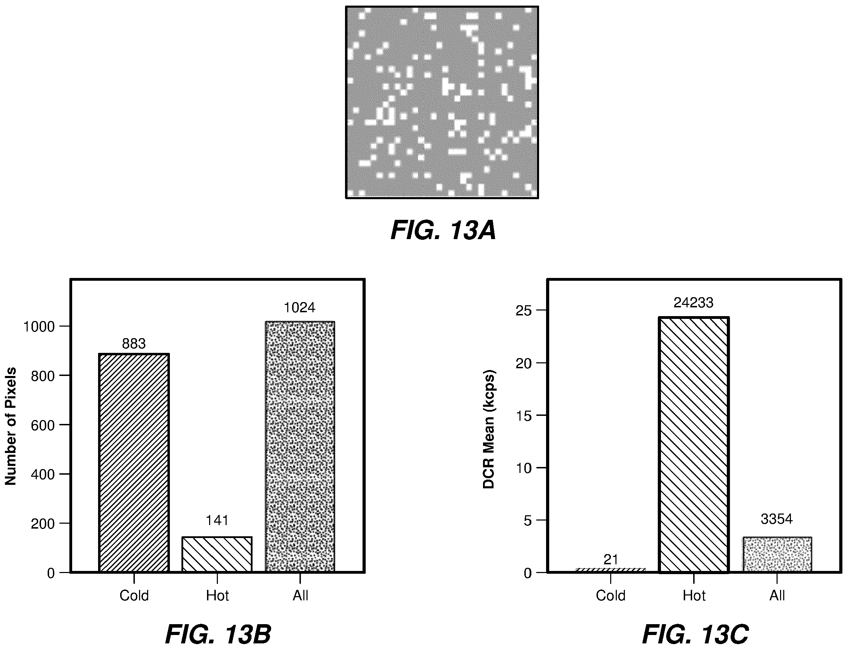

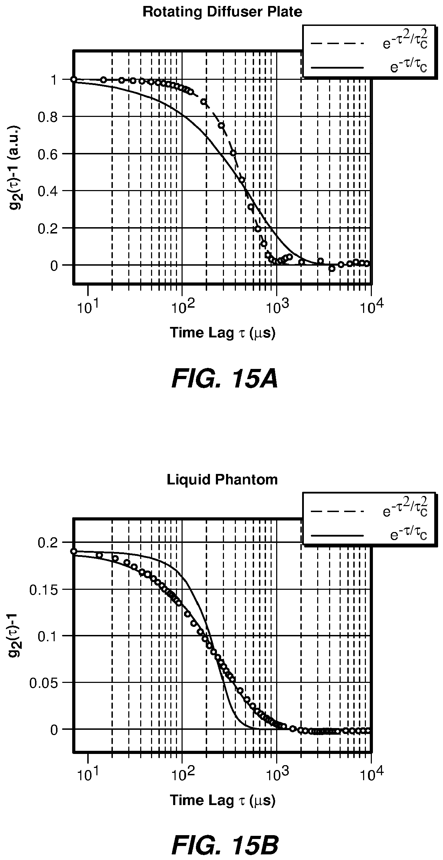

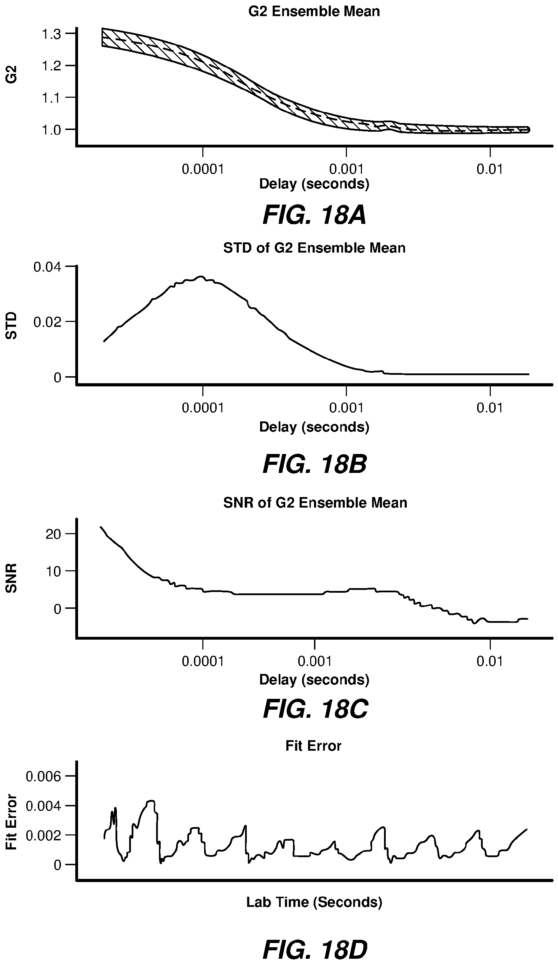

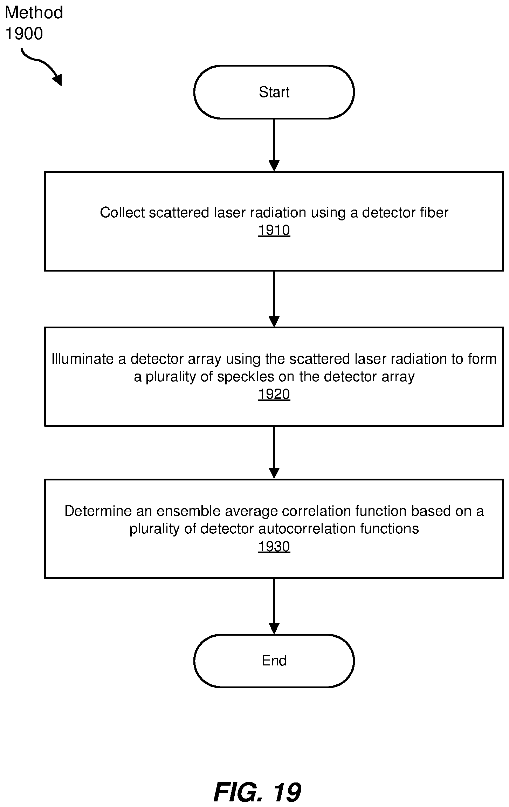

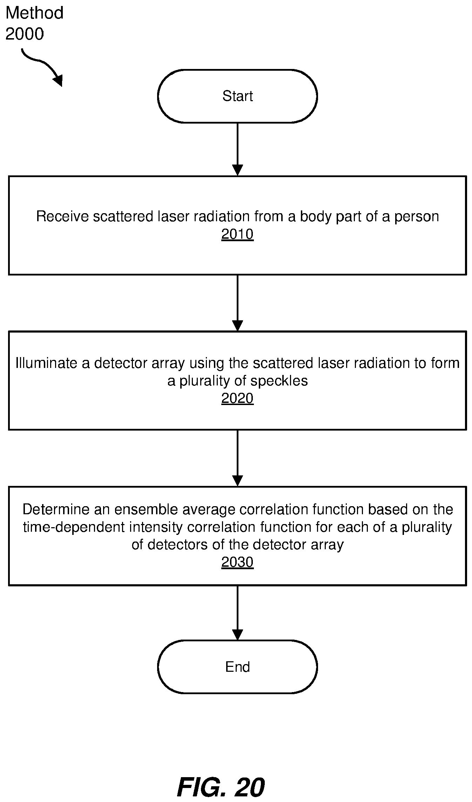

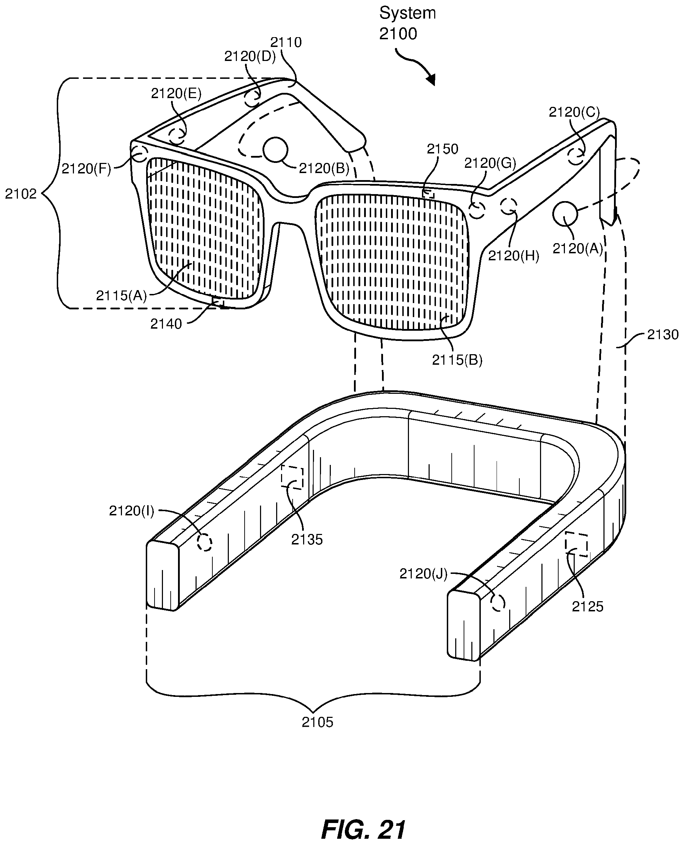

The following provides, with reference to FIGS. 1 - 22, detailed descriptions of apparatus and data analysis approaches related to multi-speckle diffuse correlation spectroscopy. FIGS. 1 - 4 show example apparatus configurations. FIGS. 5A - 6B illustrate improvements in the signal to noise ratio through formation of an ensemble average autocorrelation function. FIGS. 7A - 10C further illustrate speckle formation on a detector array and methods to characterize the diameter of the speckles. FIGS. 11A - 12B illustrate noise reduction using ensemble averaging in the formation of an autocorrelation function, and the effects of fiber core diameter. FIGS. 13A - 14C illustrate the effects of hot pixels in a detector array. FIGS. 15A - 17B show example fitting functions, the use of pulsed laser radiation, and the effects of speckle tracking using object detection. FIGS. 18A - 18J shows further example characteristics of an ensemble average autocorrelation function. FIGS. 19 and 20 are flow diagrams of example methods for fiber-based speckle contrast optical spectroscopy, and FIGS. 21 - 22 illustrate exemplary augmented reality/virtual reality applications.

Speckle correlation spectroscopy, such as diffuse correlation spectroscopy (DCS), measures the dynamics of scatterers deep within a scattering medium, such as blood-perfused tissue, by collecting the diffused laser light from the object illuminated by a source fiber and detecting the laser speckle fluctuations. The source fiber may have a source end configured to receive the laser radiation from the laser and a delivery end configured to illuminate the object with laser radiation. Since the penetration depth of laser radiation collected by the detector can be controlled by increasing the source-detector separation (p), DCS may enable the monitoring of deep tissue dynamics such as the cerebral blood flow noninvasively. However, in some implementations, the sensitivity of DCS to cerebral hemodynamics may be limited by the low photon fluxes ($N_{ph}$) detected at large p, since $N_{ph}$ decays exponentially with $\rho$. Values of $\rho$ for DCS on the adult human head may typically not exceed 25-29 mm, corresponding to a mean sensitivity depth which is roughly one-third to one-half of $\rho$ (~10 mm), which may be insufficient to effectively probe through scale and skull. To address this issue, systems and methods of the present disclosure include high-sensitivity multi-speckle DCS (mDCS), which can extend $\rho$ by detecting thousands of speckles in parallel to boost the signal-to-noise ratio (SNR).

FIG. 1 is a schematic representation of an example device 100 for fiber-based speckle contrast optical spectroscopy. The device 100 may be, for example, a medical device, wearable device, analytical instrument (e.g., a spectrometer), or other device. As shown in FIG. 1, device 100 may include a source light 110 (e.g., a laser) directing light toward a point on a user (e.g., on the user's head), illustrated by object cross-section 102. In some examples, the object 102 may represent a head. The surface of a head may be curved, but the schematic representation is simplified. The light from source light 110 may diffuse through the user's head, as represented by diffusion paths 122, 124, 126, 128, and 129. As illustrated, the greater the distance between the entry point of source light 110 on the user and an exit point of diffused light, the greater the depth reached by the diffusion path. Thus, for example, diffusion path 122 has an exit point relatively near the entry point of source light 110 and has a relatively shallow depth, while diffusion path 129 has an exit point relatively distant from the entry point of source light 110 and has a relatively greater depth. As shown in FIG. 1, device 100 may also include optical fibers (detector fibers) 132, 134, 136, 138, and 139, which may gather the light diffused through diffusion paths 122, 124, 126, 128, and 129, respectively. Device 100 may also include detector arrays (which may also be referred to as cameras) 142, 144, 146, 148, and 149, onto which light gathered by optical fibers 132, 134, 136, 138, and 139, respectively, are projected.

In some examples, an apparatus may also include an optical configuration configured to direct a portion of the unscattered laser radiation around the object. Scattered and unscattered radiation may be incident together on the detector array, and interference effects may further help increase an SNR. This is represented schematically in FIG. 1 by beam-splitter 150, which re-directs a fraction of laser radiation (before the laser radiation reaches the object, which may be termed incident laser radiation or unscattered laser radiation) as beam 152. The unscattered radiation is then recombined with scattered radiation using beam combiner 154, so that both scattered and unscattered radiation are incident on detector array (camera) 142. Other optical configurations may be used, for example, using one or more optical components such as beam-splitters, prisms, reflectors, lenses, optical fibers, apertures, and the like. In some examples, the path of the unscattered radiation may be configured so that it is approximately the same length as that of the scattered radiation. In some examples, a laser configured to provide laser radiation with a long coherence length (e.g., > 1 m, or > 10 m) may be used, as a longer coherence length may facilitate obtaining interference at the detector array.

FIG. 1 illustrates a number of aspects, one or more of which may be used in an example apparatus. For example, an apparatus may include one or more source fibers and one or more detector fibers. Different source fibers may receive laser radiation from the same laser, or, in some examples, one or more lasers may be used. Scattered laser radiation may probe dynamic processes within a scattering portion of the object, and the location of the scattering portion within the object may be adjusted by adjusting one or more of the following: the distance between the source and detector fibers, the angle of the source and/or detector fibers to the surface of the object, the wavelength of the laser radiation, or one or more other parameters that may be adjusted. For example, the depth of the scattering portion may be increased by increasing the distance between source and detector fibers. The figure shows the object as having a generally planar surface. In some examples, the object may have a curved surface (e.g., the head of a user) and one or more source fibers and/or one or more detector fibers may be arranged around the surface of the head. In some examples, a fiber may be generally normal to the local surface orientation. In some examples, the term source fiber may refer to a bundle of fibers configured to deliver laser radiation to the object, and the term detector fiber may refer to a bundle of fibers configured to collect scattered laser radiation from the object and illuminate to detector array with the scattered laser radiation. In some examples, one or more source fibers and/or one or more detector fibers may be positioned closer to a user's body in a wearable device, as discussed further below. In this context, one or more source fibers may refer to one or more fiber bundles delivering light to one or more locations on the object, and one or more source fibers may refer to one or more fiber bundles collecting light from one or more locations on the object.

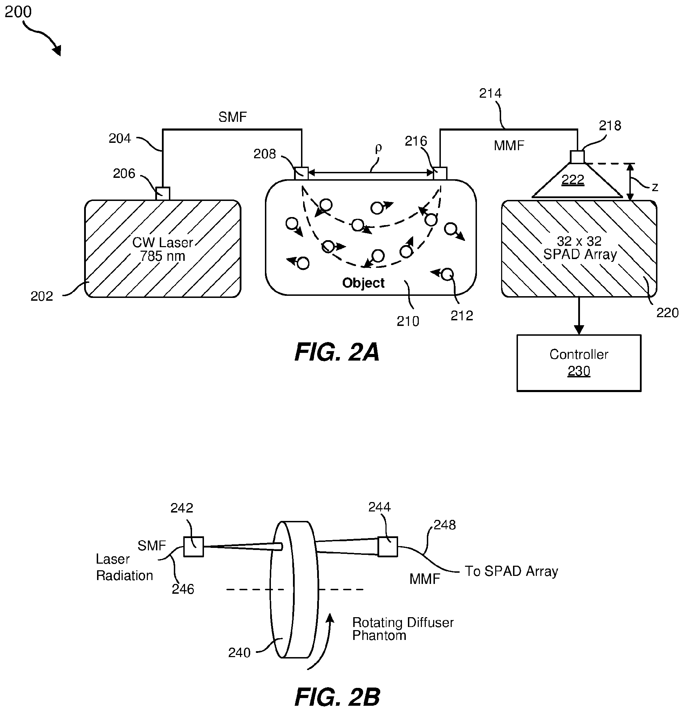

FIG. 2A shows an example apparatus 200 that may include an mDCS system based on a single-photon avalanche diode (SPAD) array. The apparatus 200 includes light source 202, source fiber 204 (e.g., a single-mode fiber) having source end 206 and delivery end 208, detector fiber 214 (e.g., a multi-mode fiber) having a collector end 216 and detector end 218, detector array 220, and controller 230. The detector array 220 may include a plurality of detectors and may, for example, include an arrangement of single-photon avalanche diodes (SPADs), such as a 32 x 32 array of SPADs. In some examples, the detector array 220 may include a SPAD array configured to detect a plurality of speckles. The object 210 is the object to be imaged, and may include object components 212 (e.g., fluid components, blood cells, or other components) engaged in a dynamic process such as diffusive motion. Scattered laser radiation, such as scattered pulsed laser radiation, emerges from the detector end 218 of the detector fiber 214 as light cone 222. The dashed lines within the object 210 approximately represents the path of incident and scattered laser light between the source fiber 204 and detector fiber 214, and this may be adjusted by adjusting the distance ($\rho$) between the source and detector fibers.

In some examples, the detector array 220 may detect 1024 speckles, or approximately one speckle per detector. This offered a 32-fold increase in signal-to-noise ratio over a single-speckle DCS. The light source 202 may be a laser, such as a long-coherence (e.g., >9 m) continuous wave (CW) laser. The laser may have an emission wavelength within visible or IR wavelengths, such as a red or near-IR wavelength. In some examples, the laser emission wavelength may in the range 700 nm - 1200 nm, for example, 700 nm - 900 nm, and example results were obtained using an emission wavelength of 785 nm. Source fiber 204 may direct laser radiation from the light source to the object 212, and the light may be coupled into the object 210 by delivery end 208 of source fiber 204. Detected light, such as scattered and/or diffused light, is collected from the object 210 by collector end 216 and directed along detector fiber 214 to detector end 218 and emerges as light cone 222 to form speckles on the receiving surface of the detector array 220. The detected light is then detected by the detector array 220, and electronic signals from the detector array 220 may be provided to the controller 230.

In some experiments, the object 210 included an equilibrium homogeneous liquid phantom, and the detector array 220 was a 32x32 SPAD array detector. The object may be any object to be imaged, such as a human head, other bodily organ or component, animal, or any other object to be imaged. The source-detector separation ($\rho$) may be adjusted, and in some experiments was set to 11 mm. The fiber-SPAD distance (z) may also be adjusted, for example, by rotating an adjustable lens tube.

Since $N_{ph}$ decays by about a factor of 10 per 10 mm, the systems and methods described herein may give a ~15 mm extension of $\rho$ and a ~6 mm increase in depth sensitivity over a single-speckle DCS. Greater improvements may also be achieved. This approach may be scalable to even more speckles (e.g., 10,000, 100,000, or more) with the use of large pixel count SPAD arrays, which may extend $\rho$ to ~50 mm or more and depth sensitivity to ~21 mm or more, reaching the cortex.

FIG. 2B shows a rotating diffuser phantom setup in which a laser (not shown) provides laser radiation to a source fiber 246, and the rotating diffuser phantom 240 is illuminated by laser radiation emerging from the delivery end 242 of the source fiber 246. Scattered laser radiation may be received by the collector end 244 of detector fiber 248 and delivered to a detector array (not shown). The rotating diffuser phantom 240 may be rotated between the fiber ends. In some configurations, an object to be imaged may be rotated (where practical) and located in place of the rotating diffuser phantom 240. The fiber ends may be arranged in a transmission geometry as shown, though other geometries may be used. This apparatus may be used to measure the diameter of the speckles.

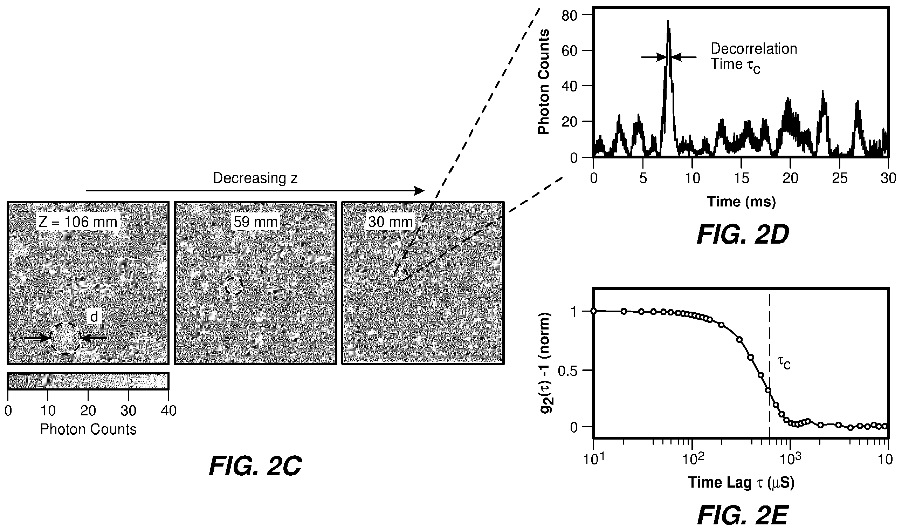

FIG. 2C shows images of speckle pattern on the SPAD array at varying distance z. Such images may be recorded using a rotating diffuser phantom to visualize the speckles at a higher photon count rate. By adjusting the fiber-SPAD distance (z = 106, 59, or 30 mm), the projected speckle diameter may be tuned (d = 8, 4, or 2 pixels). The input laser power may be adjusted to avoid saturating the detector. Replacing the phantom with a milk phantom may keep the speckle diameter unchanged, as long as the same fiber core diameter (D) and fiber-SPAD distance (z) is used.

FIG. 2D shows a photon counts time series from a single pixel as shown in FIG. 2C. This shows an example raw signal for calculating the intensity autocorrelation function $g_2(\tau)$ and the decorrelation time $\tau_c$.

FIG. 2E shows the autocorrelation function $g_2(\tau)$ of the photon counts shown in FIG. 2D.

The autocorrelation function is described in more detail below, along with an extensive discussion of the advantages of using a SPAD array.

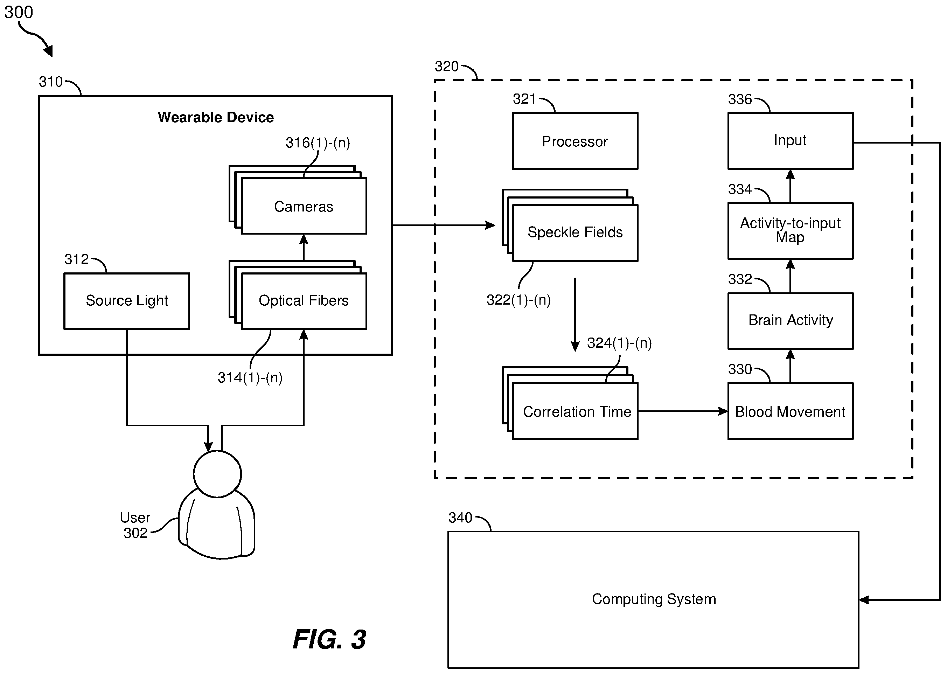

FIG. 3 is a block diagram of an example system 300 for fiber-based speckle contrast optical spectroscopy. As shown in FIG. 3, system 300 may include a wearable device 310 (worn, e.g., by a user 302). In some examples, wearable device 310 may correspond to wearable device 100 of FIG. 1. System 300 may also include a subsystem 320. Additionally, system 300 may include a computing system 340 (e.g., that receives control input). In some examples, subsystem 320 may form a part of wearable device 310. In some examples, subsystem 320 may form a part of computing system 340. In some examples, subsystem 320 may represent an intermediate subsystem separate from wearable device 310 and computing system 340.

As shown in FIG. 3, a source light 312 of wearable device 310 may direct light at user 302 (e.g., a point of the head of user 302), which light may diffuse through the blood-perfused tissue of user 302. The diffused light may exit from the blood-perfused tissue at various points, and optical fibers 314(1)-(n) may collect diffused light from some points. Optical fibers 314(1)-(n) may project the collected light onto corresponding cameras 316(1)-(n). The light projected onto cameras 316(1)-(n) may appear as speckle fields. Subsystem 320 may be configured to perform various steps executed by a processor 321. For example, subsystem 320 may receive speckle fields 322(1)-(n) from cameras 316(1)-(n). Subsystem 320 may generate contrast values (and/or autocorrelation functions) 324(1)-(n), which describe the speckle contrast (and/or the correlation times) observed in respective speckle fields 322(1)-(n). Subsystem 320 may explicitly or implicitly model, calculate, and/or derive blood movement 330 based on observed contrast values (and/or autocorrelation functions) 324(1)-(n). Subsystem 320 may then determine brain activity 332 based on blood movement 330. Subsystem 320 may apply an activity-to-input map 334 to map observed brain activity 332 to an input 336. Subsystem 320 may then provide input 336 to computing system 340, thereby performing a control operation on computing system 340 initiated by mental activity of user 302.

Contrast values may be determined in laser speckle contrast imaging. For diffuse correlation spectroscopy (DCS), the correlation time may be determined and used to estimate the speed of the blood flow. In DCS, the variation in contrast values may affect the amplitude of the autocorrelation function ($\beta$), but not the correlation time ($\tau$).

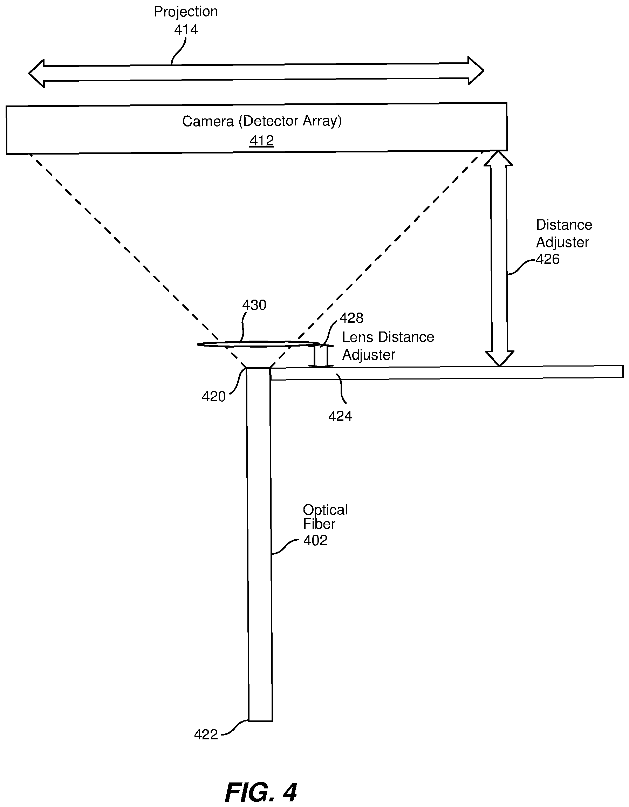

FIG. 4 illustrates a detector fiber 402 (e.g., an optical fiber) having a collector end 422 configured to receive scattered laser radiation and a detector end 420 configured to project scattered laser radiation onto a detector array 412 to form a plurality of speckles. The detector array 412 may also be referred to as a camera. The detector end 420 may be mechanically coupled to a distance adjuster 426 and may be mediated by additional optical elements, which may be used to adjust the distance between the detector end 420 of the detector fiber 402 and the detector array 412. As shown in FIG. 4, the distance between the detector fiber 402 and detector array 412 may impact the size of a projection 414 of the speckle field on the light receiving surface of detector array 412. By adjusting the distance using distance adjuster 426, the average size of speckles in the speckle field may be adjusted. The distance adjuster may include any suitable mechanical adjustment, and may include one or more of a threaded engagement, actuator, slider, or other mechanical adjustment. In some examples, a source and/or a detector fiber may be generally normal to the local surface orientation, but in some examples, the orientation of the source and/or detector fiber may be oblique (e.g., non-normal) to the local surface, and the orientation (e.g., angle relative to the local object surface normal) may be adjustable.

The configuration shown in FIG. 4 may be used to project the speckles onto the detector array and to adjust the speckle diameters using the distance adjuster. In some examples, the form factor may be reduced using one or more optical elements (e.g., lenses, filters, polarizers, splitters, or combiners) between the end of the detector optical fiber and the detector array, sometimes referred to as the camera. These optical elements can be miniaturized as needed for the application (e.g., for use in AR glasses or an AR/VR headset).

In some examples, a wearable device (such as the example device of FIG. 1) may be configured such that the average size of speckles in the speckle field is approximately one pixel. For example, the distance adjuster 426 may be adjusted so that the average size of speckles in the speckle field is approximately one pixel. In some examples, the detector end of the detector fiber 402 may include a lens that modifies the effective distance between detector end 420 and detector array 412 such that the average size of speckles in the speckle field is approximately one pixel. As used herein, "approximately" one pixel in size may indicate, for example, that the mean speckle size is between 0.5 and 2 pixels, that at least 68% of observed speckles are between 0.5 and 2 pixels in size, and/or that at least 95% of observed speckles are between 0.5 and 2 pixels in size. In some examples, the detector end 420 may be a plane surface. In some examples, detector end may include a curved surface, such as a concave surface or a convex surface. In some examples, the detector end 420 may be associated with one or more optical elements (such as an external lens) that may be adjusted to modify the speckle size. In some examples, an objective lens, adjustable tube lens, or any other suitable lens may be used. FIG. 4 shows an optional optical element, in this example lens 430 (e.g., a converging lens). The position of lens 430 may be adjustable, for example, using a threaded element (e.g., a knurled knob), slider, or any other suitable adjustment mechanism. For example, a distance between the lens 430 and the detector end 420 may be adjusted using the distance adjuster 426. The distance may be determined between the detector end 420 and the optical center of the lens 430, measured along the fiber axis and/or the optical axis of the lens (which may be parallel and may coincide).

A lens distance adjuster 428, which may be similar to and/or combined with distance adjuster 426, may be used to adjust the position of the lens 430. The lens distance adjuster 428 may be in mechanical connection with at least part of the edge portion of lens 430. In some examples, one or more of the lens 430, lens distance adjuster 428, or distance adjuster 426 may be omitted from the apparatus.

In some examples, the distance between the detector end of a detector fibers and the detector array (and/or a lens configuration associated with the optical fiber) may be fixed (e.g., such that the average size of the speckles in the speckle fields is approximately one pixel). In some examples, the distance and/or the lenses may be adjustable (e.g., such that the average size of the speckles in the speckle fields is approximately one pixel). The adjustment may be performed manually by a user and/or may be performed by the systems described herein during a calibration process (e.g., until the average speckle size is identified as approximately one pixel).

Further discussion of autocorrelation function characteristics now follows, including a detailed discussion of signal-to-noise (SNR) characteristics. When an example apparatus injects coherent light into a dynamic scattering medium (such as that shown in FIG. 2A or 2B), a dynamic speckle pattern emerges as previously shown in FIG. 2C. A DCS system may estimate the speed of the scatterers by measuring the average turnover time of the speckles ($\tau_c$), which is inversely related to the speed of the scatterers. An example apparatus may estimate a speed (or speed distribution) of the scatterers by measuring a speckle intensity vs. time and calculating the corresponding autocorrelation function ($g_2$), for example, as previously illustrated in FIG. 2D and 2E.

Even though the SNR of $g_2$ may be increased by having longer integration times (the time over which the speckle intensity is recorded for each $g_2$ calculation, $T_{int}$) and higher photon count rates ($N_{ph}$), these parameters are limited by the time scales of the dynamics used to measure and the laser maximum permissible exposure (MPE) on skin. In one approach, a single-mode fiber (SMF) may be used as the detection fiber to ensure coupling of only one speckle onto a single-photon detector (called "single-speckle DCS"). In another approach, a multi-mode fiber (MMF) may be used to couple multiple (M) speckles onto the single-photon detector. However, although the detection count rate increases ($N_{ph} \propto M$), the magnitude of $g_2$ decreases with the number of speckles arriving at the detector ($\beta \propto 1/M$), effectively resulting in no gain of the $g_2$ SNR.

Multi-speckle DCS (mDCS) allows significant improvements in the SNR, compared to single-speckle DCS, using parallel DCS measurements of M > 1 speckles to provide M independent photon-counting channels. Parallel DCS measurements of M = 1024 speckles may be achieved by using a kilopixel SPAD array. While coupling one speckle on every pixel in the SPAD array gave an SNR gain of 32x, pulsing a CW laser source may give an additional SNR gain. For example, an experimentally obtained SNR gain of 3.2x from using pulsed laser radiation resulted in a total SNR gain of more than 100. The additional SNR gain due to pulsing the laser may be increased by reducing the duty cycle of the laser. For example, the SNR gain may be related to the inverse of the square-root of the duty cycle. If the duty cycle is 1, the SNR gain may be 1 (corresponding to no SNR improvement). If the duty cycle is 0.25, then the SNR gain may be 2. The duty cycle may be adjusted to obtain a desired SNR gain. Systems and methods implementing this approach (e.g., pulsed laser operation) may provide a scalable implementation of DCS that allows both high SNR and high sensitivity to the cortex.

An example apparatus may include a laser, such as a semiconductor laser, such as a visible (e.g., red-emitting) or near-IR CW laser diode. In some experiments, a 780 nm CW laser may be coupled to an object using a source optical fiber (such as a single-mode fiber). The object may include a dynamic scattering medium. A detector optical fiber (e.g., a multimode fiber) may be used to direct scattered light to a detector, such as a SPAD array. The scattered light may include light diffusing out of the scattering medium. In some experiments, for evaluation purposes, the object may include an equilibrium homogeneous liquid phantom. For example, a phantom may be used to determine the SNR for a particular apparatus configuration. In some examples, the object may be a static or a rotating diffuser, which may be used to determine the diameter of the speckles.

In some examples, the detector array may include 1024 SPADs arranged in a 32x32 array, with a pixel pitch of 50 x 50 $\mu$m and an active area of 6.95 $\mu$m in diameter at each pixel. In some examples, the pixels may be generally square pixels, but other pixel shapes are possible, such as circles. Each pixel may have its own photon counting electronics, for example, that may run at greater than 250,000 frames per second. Operation at 625 kfps was obtained using a 32 x 32 detector array (in this example, a SPAD detector array that may be referred to as a SPAD camera or SPAD array) The SPAD detector array enables simultaneous detection of a plurality of speckles, and this results in an appreciable SNR improvement of the autocorrelation function($g_2$). In some examples, an apparatus may ensure that each SPAD pixel detects one or more speckles by adjusting the speckle size to be equal or smaller than the pixel active area.

The average diameter of a speckle ($d$) obeys the relationship of Equation (1):

$$d = \lambda z / D \qquad \text{(Equation 1)}$$

Here, A is the wavelength of the light (785 nm), *z* is the distance between the detection fiber and the SPAD array (3.5-10 mm) and *D* is the fiber core diameter (e.g., 200-910 μm). In some examples, apparatus configurations may allow a reduction in *d* by decreasing *z*, and/or by using a larger fiber core diameter *D*.

A high-contrast image of the speckles may be formed on the SPAD array, and the diameter of the speckles at varying distance z may be determined. This may use a high-throughput rotating diffuser phantom, such as shown in FIG. 2B, with source (SMF) and detection (MMF) fiber ends arranged in the transmission geometry shown in FIG. 2B. FIG. 2C shows static images of the speckles illustrating that the speckle diameter (d) can be made smaller by decreasing z. As the diffuser phantom is rotating, the time trace of the photon counts on each pixel can be recorded, and $g_2$ can be calculated (as previously illustrated in FIG. 2E). In this way the measured $g_2$ curves and their SNRs for several values of *d* may be compared.

In other experiments, an equilibrium milk phantom in a reflection geometry with a $\rho$ of 11 mm may be used in a configuration similar to that shown above in FIG. 2A. A milk-based phantom may better represent the fast decorrelation time (≤ 200 μs) and low photon count rate seen in typical human tissue studies. The condition of one speckle per pixel active area may be satisfied using a fiber core diameter of 910 μm and a fiber-SPAD distance of 8.1 mm. Other configurations may be used. In these measurement, the SPAD camera may record photon counting time traces on every pixel with a frame exposure time of 4 μs ($T_{bin}$) for up to 2 million frames (8.0 seconds).

From each pixel (e.g., each detector of a detector array), the $g_2$ function as a function of time lag $\tau$ may be calculated. Then a comparison may be made with the $g_2$ curve obtained in a single run from a single pixel ("the pixel $g_2$") as in Equation 2:

$$g_2^i(\tau) = \frac{\langle n(t)n(t+\tau)\rangle}{\langle n(t)\rangle\langle n(t+\tau)\rangle} \qquad \text{(Equation 2)}$$

The curve obtained from the ensemble average of all 1024 pixels ("the ensemble $g_2$") is given by Equation 3:

$$\bar{g}_2(\tau) = \frac{1}{M}\sum_i^M g_2^i(\tau) \qquad \text{(Equation 3)}$$

Here, *n*(*t*) is the number of photons counts in time bin *t,* the angle bracket (...) denotes the average over an integration time $T_{int}$, and M is the number of independent speckle field measurements.

FIGS. 5A - 5C show measurements of *R* = 160 runs with an integration time of $T_{int}$ = 50 ms in each run, calculating the time statistics over all runs. A symmetric normalization may be adopted in these calculations.

FIG. 5A shows the time statistics (mean and standard deviation) of a pixel $g_2$.

FIG. 5B shows the time statistics for an ensemble $g_2$. As can be seen from a comparison of FIGS. 5A and 5B, the standard deviation (STD) is significantly reduced for the ensemble average autocorrelation function $g_2$.

FIG. 5C shows the measured signal-to-noise (SNR) ratio ($g_2$/STD) at increasing ensemble size (shown as points) accompanied by the calculated SNR from the mDCS noise model (shown as continuous lines) discussed further below. The SNR shows an overall increase with larger ensemble size, which matches the theoretical predictions. The SNR gain (comparing the ensemble $g_2$ and the pixel $g_2$) is calculated by taking the ratio of the measured ensemble $g_2$ to the pixel $g_2$ of the first bin ($\tau$ = 4 μs).

FIG. 5D shows that the measured SNR gain increases as $\sqrt{M}$ increases and reaches a maximum value of 32 when the full array is used.

The SNR of $g_2$ in single-speckle DCS is determined by a number of parameters that includes bin time, integration time, count rate, decorrelation time, and coherence factor. In order to evaluate mDCS, a noise model that may be applied to single-speckle DCS may be extended to mDCS. Under the assumption of ergodicity, this may be accomplished by incorporating the number of speckles together with the integration time in the model. In this new model, the $g_2$ STD at each time lag may be estimated according to Equation 4 below:

$$\sigma(\tau) = \sqrt{\frac{T}{tM}\left[\beta^2 * \frac{(1+e^{-2\Gamma T})(1+e^{-2\Gamma\tau}) + 2m(1-e^{-2\Gamma T})(e^{-2\Gamma\tau})}{(1-e^{-2\Gamma T})} + 2\langle n\rangle^{-1}\beta(1+e^{-2\Gamma\tau}) + \langle n\rangle^{-2}(1+\beta e^{-2\Gamma\tau})\right]}^{1/2}$$

(Equation 4)

Here, $T$ (= $T_{bin}$) is the correlator time bin interval, $t$ (= $T_{int}$) is the averaging integration time, M is the number of detected speckles, $\beta$ is the coherence factor $g_2(0)$, $2\Gamma$ is the decay rate, m is the delay time bin index, and $<n>$ (= $N_{ph}$ X $T_{bin}$) is the count rate within $T_{bin}$ per pixel. The multi-speckle factor M plays the same role statistically with $T_{int}$. This results from the ergodicity of a random process in the system. To validate the mDCS noise model, a comparison of the measured $g_2$SNR at increasing $T_{int}$ or $N_{ph}$ to increasing the ensemble size M at short $T_{int}$ or low $N_{ph}$ is performed.

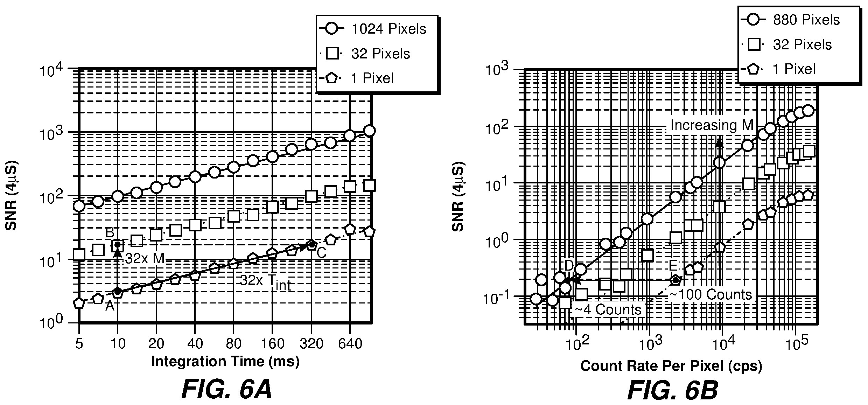

FIGS. 6A and 6B shows SNR improvements as a function of integration time and count rate per pixel, respectively.

FIG. 6A shows the SNR comparison at increasing integration time ($T_{int}$) with different numbers of individual detectors, sometimes referred to as pixels (where M = 1, 32, or 1024). By increasing the ensemble size at short $T_{int}$ (10 ms) the same SNR gain is obtained that would be achieved by one pixel at $10^3$ times longer $T_{int}$ (10 s).

FIG. 6B shows the SNR comparison at increasing count rate by increasing the laser input power, and demonstrates that increasing the ensemble size is equivalent to increasing the count rate of one pixel by 30 times. As can be seen from these plots, the mDCS noise model (in solid line) is in close agreement with the experimental results (shown as points), even in the absence of any fitting parameters. In particular, there is a linear dependence of the SNR with respect to $N_{ph}$ in the low count rate regime. This is well predicted by the mDCS noise model (Equation 4), which is dominated by the third term in the low count rate limit and approximated as in Equation (5) below:

$$\sigma(0) = \frac{1}{\langle n\rangle}\sqrt{\frac{T}{tM}(1+\beta)} \qquad \text{(Equation 5)}$$

This leads directly to Equation (6):

$$\text{SNR}(0) = \langle n\rangle\sqrt{\frac{tM}{T}\frac{\beta^2}{(1+\beta)}} \qquad \text{(Equation 6)}$$

The square-root dependence of SNR(0) to $t$ (= $T_{int}$) and M arises from the ergodicity of the measured system. In this way, a higher SNR beyond the $T_{int}$ and $N_{ph}$ limits is achieved by using larger M. DCS measurements may be configured to detect tissue dynamics at longer $\rho$ (or larger penetration depths) at the expense of count rates. Therefore, this comparison validates the mDCS noise model in the low count rate regime. The ensemble averaging allows the recovery of some gains of the SNR even when the total count rate is approaching the SPAD's dark count rate (DCR) of < 100 Hz per pixel.

FIG. 6B further illustrates how the ensemble averaging technique is applicable to photon-starved applications, such as the detection of deep-tissue dynamics. FIG. 6B shows that when the photon flux was as low as ~4 counts per pixel per $T_{int}$ (point D), ensemble averaging resulted in the same SNR as with only one pixel with ~100 counts per $T_{int}$ (point E). mDCS reached the predicted SNR gain even in the case of an unprecedentedly small number of counts available to calculate $g_2$ in each pixel.

In addition to capturing more speckles, the SNR may be further increased by pulsing the laser at higher peak power ($N_{ph}$) at the expense of shorter pulse width ($T_{int}$), as long as the average power is below the MPE on skin. This approach results in a net SNR gain because the SNR is linear in $N_{ph}$ and square-root in $T_{int}$. This approach may be validated by comparing the usual SNR from using a CW laser to the SNR that would be obtained from 15x laser input peak power at 1/15 duty cycle, which results in a 3.5x SNR gain. The combination of this pulsed mode (3.5x SNR gain) with the kilopixel SPAD array (32x SNR gain) leads to a total of >100x SNR gain.

Accordingly, examples of the present disclosure include a scalable approach for mDCS using a kilopixel SPAD array, and with a pulsed mode, to enhance the SNR gain by a factor of greater than 100 times compared to the single-speckle CW-DCS. This means that this technique may be used to measure signal changes on significantly faster time scales and/or longer penetration depth. Thus, if a conventional technique would allow a $\rho$ for DCS measurement on the adult human head as high as 29 mm using M = 14 channels, the techniques discussed herein may allow an increase of $\rho$ by ~15 mm or more and an increase in depth sensitivity by about ~6 mm or more. A noise model for mDCS may be established by assuming speckle ergodicity (which assumptions are supported quantitatively with experimental results), where the SNR is approximately proportional to $N_{ph} \times \sqrt{T_{int}} \times \sqrt{M}$ in the shot noise limit. In addition, the mDCS model may be applied in the low photon limit. The kilopixel SPAD array offers a significant increase of channels (M = 1024) by a factor of 36 times as compared to M = 4 - 28.

With the advent of LIDAR technology, high-sensitivity kilopixel SPAD arrays with small dark count rates and high frame rates are commercially available, and detector arrays having larger numbers of pixels may be fabricated. This allows further increases in SNR using SPAD arrays having greater than at least 10,000, or at least 100,000, and in some examples at least 1 million pixels. Using a larger number of pixels, a larger fiber core diameter may be used to accommodate more speckles and faster data transfer and processing rates for real-time mDCS measurements. As is explained in greater detail below, this technique can also be implemented in the time-domain mDCS to enable enhanced depth sensitivity.

The approaches described herein for mDCS can also be implemented in the time-domain to enhance sensitivity to deep tissue. As discussed above, the steady-state operation of mDCS may use a continuous wave (CW) laser light source. A challenge with steady-state DCS measurements is that the total signal includes the desired signals from deep tissues (e.g., the brain) in addition to the unwanted signals from the intervening superficial tissues (e.g., the scalp and skull). This problem occurs because, in steady-state DCS, all photons are detected from the source point which reaches the detector point regardless of the path the photons took through the tissue. Employing pulsed laser light source, improved multi-speckle time-domain diffuse correlation spectroscopy (mTD-DCS) may be achieved. The time-domain approach enables systems and methods described herein to selectively capture different photons that have traveled along different path lengths through tissue. As a rule of thumb, photons are injected from the source point and capture the returning photons at the detector point, and photons that have traveled through deep tissue have longer path lengths as compared to photons that have traveled through superficial tissue. In other words, photons that have traveled through deep tissues may arrive a few hundreds of picoseconds to a few nanoseconds later as compared to the photons that have only traveled through superficial tissues. By applying time-gated strategies to the DCS signal, systems described herein can differentiate between short and long photon path lengths through the tissue and determine the tissue dynamics for different depths. This technique involves picosecond pulsed laser sources, and a time-correlated single-photon counting (TCSPC) to time-tag each detected photons with two values, the time-of-flight from the source to the detector points to obtain the temporal point-spread function (TPSF), and the absolute arrival time to calculate the temporal autocorrelation function for DCS. By evaluating the correlation functions over different time gates of the TPSF, TD-DCS may differentiate between early and late arriving photons and evaluate the dynamics at different depths within the tissue. The mDCS approach described herein using a kilopixel to megapixel SPAD arrays may enable parallel independent measurements of TD-DCS signals and further increase the instrument sensitivity.

The number of speckles that are projected on a SPAD array may determine the maximum SNR enhancement factor that is achieved with the mDCS technique. However, the instantaneous number of speckles may change over time because the speckles from dynamical scattering media are constantly changing over time (i.e., appearing and disappearing across time). Accordingly, an object detection technique to locate every speckle and count the number of speckles per frame is used. The number of speckles per frame increases as the speckle size decreases.

An example configuration may use a rotating diffuser phantom in transmission geometry with a 785 nm CW laser source, a SMF source fiber end (4.4 $\mu$m core diameter, 0.13 NA), and a MMF detector fiber end (400 $\mu$m core diameter, 0.5 NA). The speckle diameter and the number of speckles can be adjusted by tuning the fiber-SPAD distance.

FIG. 7A shows the speckle pattern on an example SPAD array with about 21 speckles identified, and each speckle is about 5 pixels in diameter. The time statistics of the number of speckles per frame can be obtained by recording the image and performing the speckle tracking on every frame.

FIG. 7B shows the time trace of the number of speckles with 10 $\mu$s frame exposure time for up to 500 frames. The speckle tracking detects 21 speckles on the frame by identifying their peak intensity characteristics across the array, and each speckle is about 5 pixels in diameter. The speckle turnover time is about 1 ms, adjustable by the rotation speed. FIG. 7B shows the time trace of the number of speckles per frame at fiber-SPAD distance of 82 mm (solid line) and 158 mm (dashed line). As shown in the figure, the number of speckles per frame varies over time.

FIG. 7C shows the histogram of the number of speckles over time around the mean value of 22 (upper histogram) and 12 (lower histogram) speckles per frame. This speckle tracking technique alone may fail when the speckle size is too small (comparable with the pixel size) or too large (comparable with the camera size). If the speckle size becomes too large, the diameter can be measured using a 2D autocorrelation technique. If the speckle size becomes too small, its diameter may be estimated using a pixel clustering method.

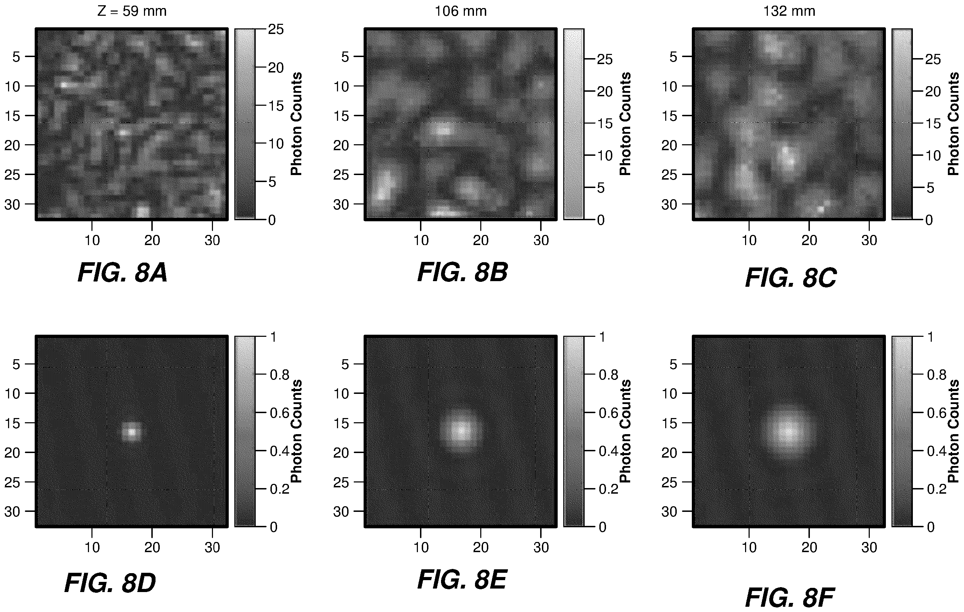

FIGS. 8A - 8H illustrate speckle formation on a detector array, and the effects of adjusting the distance between the detector end of the fiber and the detector array.

FIGS. 8A - 8C show that the speckle size gets larger by increasing the fiber to detector array distance (z). In these examples, a detection fiber core diameter of 400 $\mu$m and a numerical aperture of 0.50 may be used.

FIGS. 8D - 8F show that, by calculating the 2D autocorrelation function of each multi-speckle image, the average speckle size may be quantified.

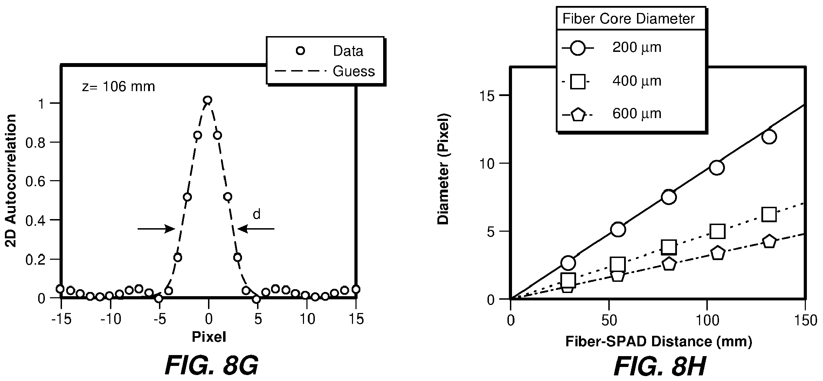

FIG. 8G shows the linecut of the 2D spatial autocorrelation image at z = 106 mm.

FIG. 8H shows that the diameter of the speckles scales linearly with $z$, which can be expressed as $d = \lambda z/D$, where $d$ is the average diameter of the speckles, A is the laser wavelength, $z$ is the fiber-SPAD distance, and D is the detection (MMF) fiber core diameter.

A pixel clustering method may be used to determine the diameter of the speckles. This method may use the decrease in magnitude of the coherence factor$\beta$ as the photon counts from more speckles are summed up prior to calculating $g_2$.

An example apparatus may include a rotating diffuser phantom in transmission geometry with a 785 nm CW laser source, a SMF source fiber end (4.4 $\mu$m core diameter, 0.13 NA), and an MMF detector fiber end (400 $\mu$m core diameter, 0.5 NA). The speckle diameter and the number of speckles can be adjusted by tuning the fiber-SPAD distance.

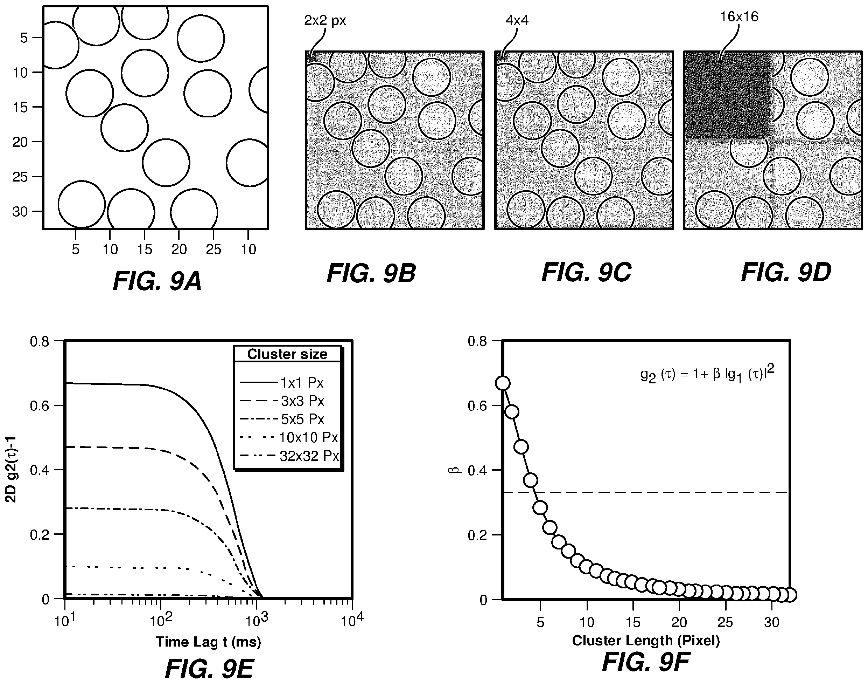

FIGS. 9A - 9F illustrate cluster sizes for projections of speckles onto a detector array.

FIG. 9A shows that multiple speckles may be projected on a SPAD array, where the speckle diameter may span more than one pixel. One or both of two methods may be used to determine the temporal autocorrelation function $g_2$ from this type of image data. One method is to calculate $g_2$ from each pixel, where 1024 x $g_2$ is obtained from each repetition. Another method is to first perform a pixel clustering such that the size of each cluster is comparable to the speckle size and then calculate $g_2$ from each cluster.

FIGS. 9B - 9D show that, for example, with 2x2, 4x4, or 16x16 pixels in each clusters (respectively), and 256x, 64x, or 4x $g_2$ is obtained from each repetition. For example, in a more accurate representation, the shaded region in the 4x4 case may be twice as large as the shaded region in the 2x2 case.

FIG. 9E shows that the magnitude of the $g_2$ clusters may decrease as more than one speckle per cluster is detected.

FIG. 9F shows that the number of speckles per cluster may be determined form the coherence factor ($\beta$) that appears in the Siegert relation for multimode detection $\beta = \frac{\beta_{max}}{K}$, where $\beta_{max}$ = 0.67 in this configuration and $K$ is the number of speckles per cluster. $\beta$ decreases as the cluster length is increased. The crossing at $\beta = \frac{\beta_{max}}{2}$ corresponds to the cluster length where two speckles per cluster are detected.

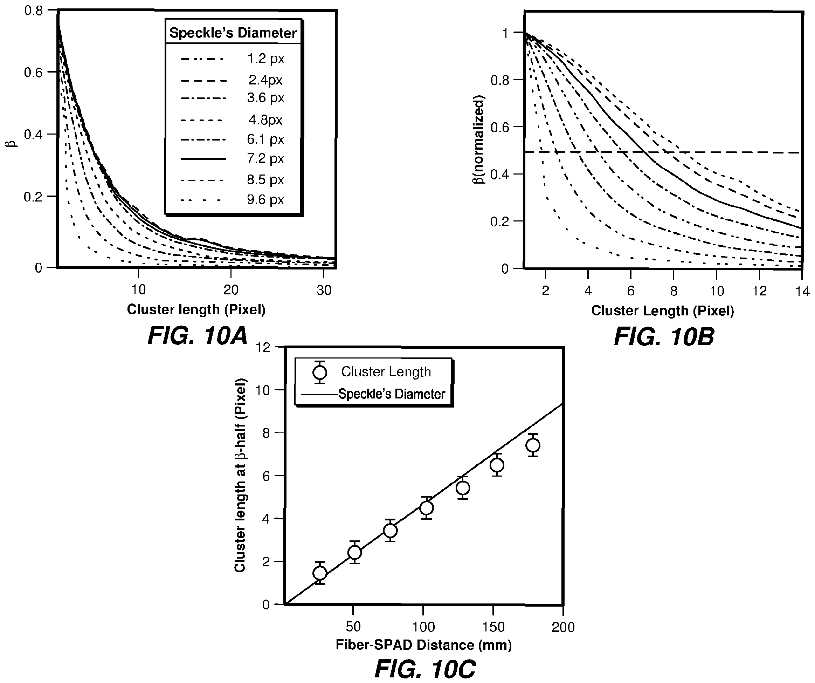

FIGS. 10A - 10C show that the cluster length (obtained using the method discussed above in relation to FIG. 9F) is equal to the speckle diameter even when the speckle diameter is increased. FIG. 10A shows the relationship of coherence length ($\beta$) to cluster length in pixels. FIG. 10B shows the relationship of coherence length ($\beta$, normalized to 1) to cluster length in pixels. FIG. 10C shows the cluster length at half-$\beta_{max}$ to fiber-SPAD distance in millimeters, showing an approximately linear relationship.

FIGS. 11A-11F illustrate cluster and ensemble averaging in formation of an autocorrelation function, with reduced signal-to-noise for ensemble averaging.

FIG. 11A illustrates how, at every repetition R, $g_2$ may be calculated from each cluster (called a "$g_2$ cluster"). Individual clusters may be represented by squares, such as squares 1100. Multiple measurements may be summed for individual clusters. The second layer 1102 represents a second repetition. FIG. 11B shows how the ensemble average of all $g_2$ clusters at every repetition R can also be calculated (called a "$g_2$ ensemble"). This is represented by the shaded area 1100, covering a plurality of clusters (e.g., all clusters) as represented by the ensemble of individual squares. In these examples, measurements up to R = 19 repetitions were taken ($T_{int}$ = 65 ms in each repetition), where the mean $g_2$ cluster and its standard deviation over all repetitions is obtained to calculate the SNR of the cluster. Similarly for the ensemble, the mean $g_2$ ensemble and its standard deviation may be obtained over all repetition to calculate the SNR of the ensemble.

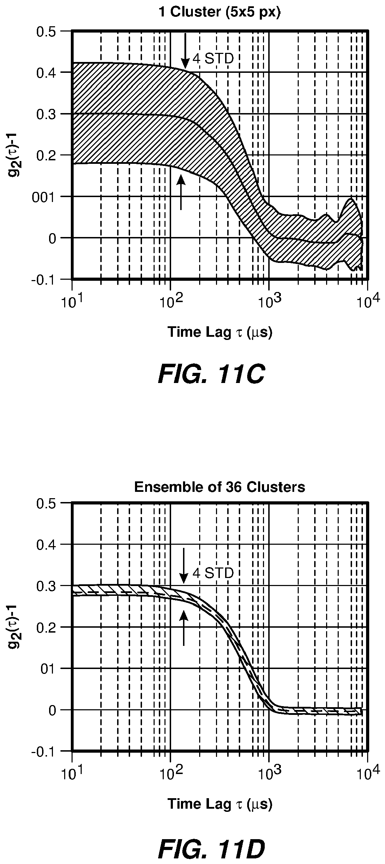

FIGS. 11C and 11D show that the SNR for the ensemble average is higher than the SNR for the cluster average, showing the advantage of ensemble averaging. FIG. 11D shows results for a cluster ensemble, showing an SNR gain of 6 times relative to the results for a single cluster shown in FIG. 11C. The hashed region around an average curve is representative of the noise content. The SNR improvement is because the ensemble includes 6x6 clusters, whereas each cluster (5x5 pixels) is adjusted to the speckle diameter (5x5 pixels).

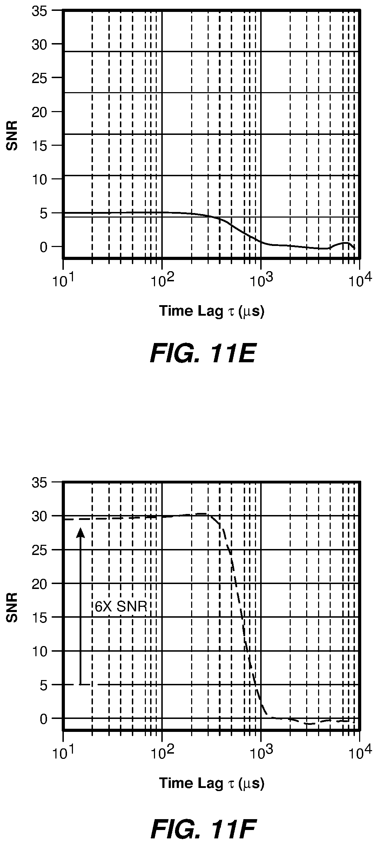

FIGS. 11E and 11F show SNR as a function of time lag in microseconds. SNR enhancements are greater for shorter time lags.

A pixel clustering method may determine the speckle size, or the number of speckles, even without having a good speckle visualization, which is beyond the capability of conventional techniques.

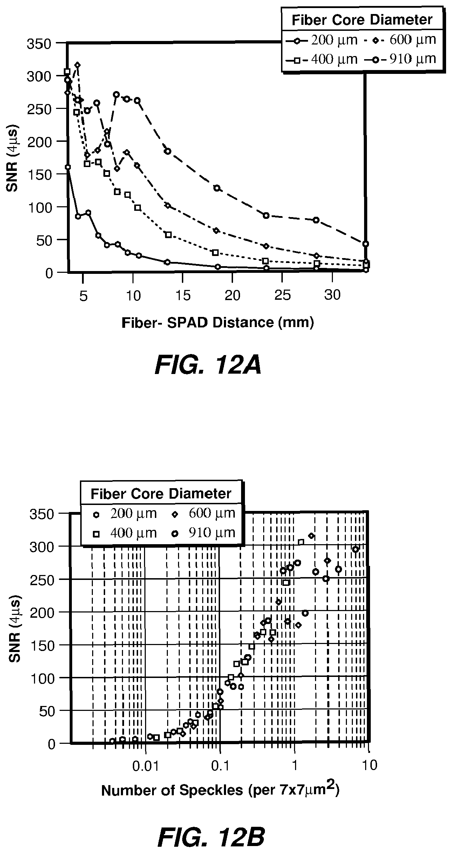

FIGS. 12A and 12B illustrate the effect of fiber core diameter on SNR.

FIG. 12A shows the variation of SNR with the fiber-SPAD distance for different fiber core diameters. The fiber core diameter may determine the speckle size on the SPAD pixel, and different fiber core diameters (and, accordingly, different speckle sizes) may affect the ensemble SNR. Larger fiber core diameters may produce significantly higher SNR at fiber-SPAD distance of z = 5 mm or shorter (as shown in FIG. 12A) and lower SNR at longer z.

FIG. 12B shows that the maximum SNR can be achieved if the speckle diameter is adjusted to be comparable to the pixel active area (e.g., 6.95 $\mu$m in diameter), which is smaller than the pixel pitch (50 x 50 $\mu$m). This can be investigated by plotting the ensemble SNR (from all fiber diameters) against the number of speckles per pixel active area, as shown in FIG. 12B. As seen in FIG. 12B, all of the data points fall onto a single curve, suggesting that, for a given laser input power ($N_{ph}$) and integration period ($T_{int}$), the SNR of mDCS depends solely on the number of speckles per pixel active area. The SNR may keep increasing until it reaches one or more speckles per pixel active area (as shown in FIG. 12B). While $\beta$ decreases as 1024/M, STD also decreases as M/1024 due to more photon counts $N_{ph}$ from each speckle, thus keeping the SNR the same at M > 1 per pixel active area.

Physical defects in SPAD pixels can effectively increase the dark count rate (DCR) due to trapped carriers and after-pulsing. Pixels with a high DCR (>100 Hz) may be referred to as "hot pixels" and pixels with a low DCR (<100 Hz) may be referred to as "cold pixels".

FIGS. 13A - 13E show the effects of hot pixels in a kilopixel SPAD array on the intensity autocorrelation function $g_2$ and SNR.

FIGS. 13A - 13B shows the pixel distribution and the corresponding histogram of 141 hot pixels (lighter) and 883 cold pixels (darker), determined from the count rate while the camera aperture was covered. The hot pixels make up less than 14% of the total number pixels.

FIG. 13C shows that the mean DCR (dark count rate) of hot pixels is 24.2 kcps (thousands of counts per second) and for cold pixels is 21 cps, with a mean DCR of 3.3 kcps across all pixels. The DCR has an approximately binary distribution, being either very low or very high.

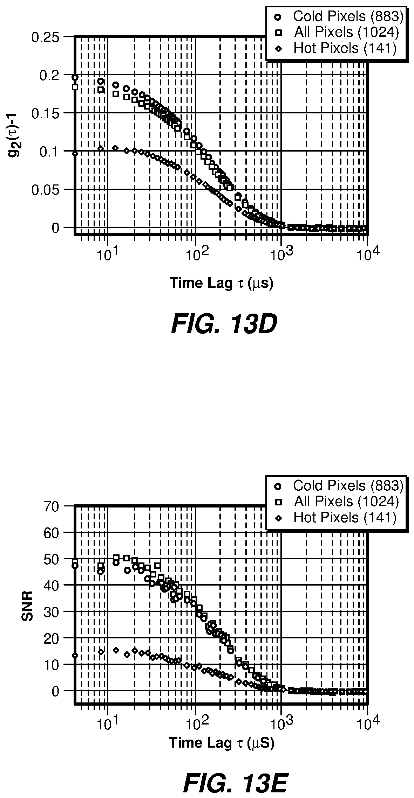

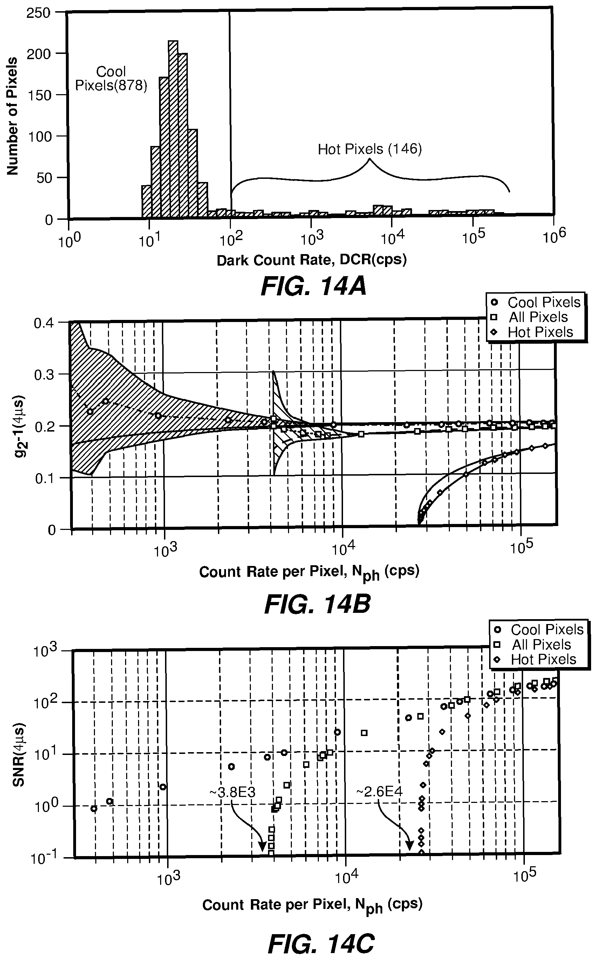

FIGS. 13D - 13E show that the ensemble average of 883 cold pixels gives a slightly higher $g_2$ value as compared to the ensemble average of all 1024 pixels, while the ensemble average of 141 hot pixels gives much lower $g_2$ and SNR values as compared to the rest. The SNR and $g_2$ is calculated from all 1024 pixels and the ensemble average is taken across selected pixels (using $T_{int}$ = 50 ms, over 160 repetitions, and 5 mW input power). Although in this example of high count rate the ensemble of cold pixels does not give a higher SNR as compared to all pixels (as shown in FIG. 13E), the count rate accumulated in all pixels includes a large false count rate (DCR) contribution from the hot pixels. In situation of low count rate the ensemble of cold pixels does give a higher SNR as compared to all pixels (as shown in FIG 14C).

FIGS. 14A - 14C further illustrate characterization of hot pixels in a 32 x 32 kilopixel SPAD array. Cool pixels have a DCR < 100 Hz and hot pixels have DCR > 100 Hz. The SPAD camera aperture was covered with a blank.

FIG. 14A show a distribution of the DCR across all pixels. There were 878 cool pixels and 146 hot pixels. The mean DCR of cool pixels was 24 cps (counts per second) for cool pixels, 3806 for all pixels, and 26.6 kcps for hot pixels.

FIG. 14B shows the measured $g_2$ - 1, corresponding to coherence factor $\beta$, across selected pixels at varying count rate per pixel ($N_{ph}$). A liquid phantom was used with source-detector separation of 11 mm and a 785 nm CW laser. The standard deviations are shown by the widths of the shaded regions. Solid circles show the measured values of $g_2$ - 1, and solid lines show the predicted values based on calculations.

FIG. 14C shows the measured SNR of $g_2$ across selected pixels at varying $N_{ph}$ (over 160 integration periods).

FIGS. 15A and 15B show example fitting functions for an intensity autocorrelation function ($g_2(\tau)$).

FIG. 15A shows normalized values of ($g_2(\tau)$ - 1) (solid circles) as a function of time lag ($\tau$) measured from the rotating diffuser plate, along with two different functional forms of $g_2(\tau)$ - 1 that were used to fit the measured data. The solid line shows a fit including a term $\exp(-\tau/-\tau_c)$, which does not fit the data particularly well. The dashed line shows a better fit including a term $\exp(-\tau^2/\tau_c^2)$. The $g_2$ curve was normalized by the value of $g_2$ at $\tau$ = 10 $\mu$s. The measured data can be fitted well using a fit of the form $g_2(\tau) - 1 = \beta \exp(-\tau^2/\tau_c^2)$, where $\tau_c$ is 557 $\mu$sec, matching the ballistic motion dynamics of the rotating diffuser plate. A measurement of $\beta$ = 0.7 was obtained with the rotating diffuser plate setup. The resulting $\beta$ was higher than the value expected from the case of unpolarized light ($\beta$ = 0.5), possibly due to a polarization bias in the detected light. The speckle diameter may be adjusted to be three times larger than the pixel size ($d > 3a$). In some examples, the speckle diameter may be adjusted to be within 1 - 5 times larger than a pixel dimension (e.g., a pixel edge length or diameter), for example, in the range 2 - 4 times larger.

FIG. 15B shows $g_2(\tau)$ -1 (solid circles) measured from a liquid phantom and two functional forms curve fitting (solid curves), similar to those discussed above in relation to FIG. 15A. The measured $g_2(\tau)$ may be fitted using a function of the form $g_2(\tau) - 1 = \beta \exp(-\tau/-\tau_c)$, giving a value $\tau_c$ of 197 µs, matching the diffusive motion dynamics of the liquid phantom. In this example, the liquid phantom setup resulted in a measurement of $\beta = 0.2$. The resulting $\beta$ was lower than the value expected from the case of unpolarized light ($\beta = 0.5$) possibly due to the speckle diameter being approximately matched with the pixel active area (d = a). The maximum value for unpolarized light ($\beta = 0.5$) may be obtained by increasing the speckle diameter beyond the Nyquist rate (d > 2a).

As discussed earlier, SNR is approximately proportional to $N_{ph}$ x $\sqrt{T_{\text{int}}} \times \sqrt{M}$ in the shot noise limit. Furthermore, $N_{ph}$ has an upper limit that is determined by the MPE for skin exposure, thus limiting the SNR. Although this limit can be overcome through capturing more speckles, the SNR can be increased further by changing the parameter combination between $N_{ph}$ and $T_{int}$. This is because the two parameters have different exponents (linear in $N_{ph}$ and square-root in $T_{int}$). In this way, $N_{ph}$ can be increased for a shorter effective $T_{int}$ without going beyond the average power set by MPE.

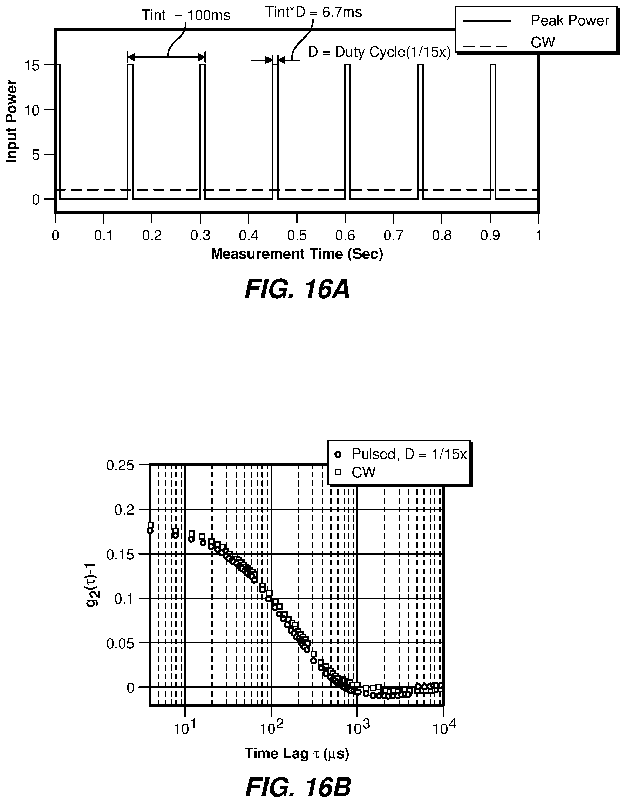

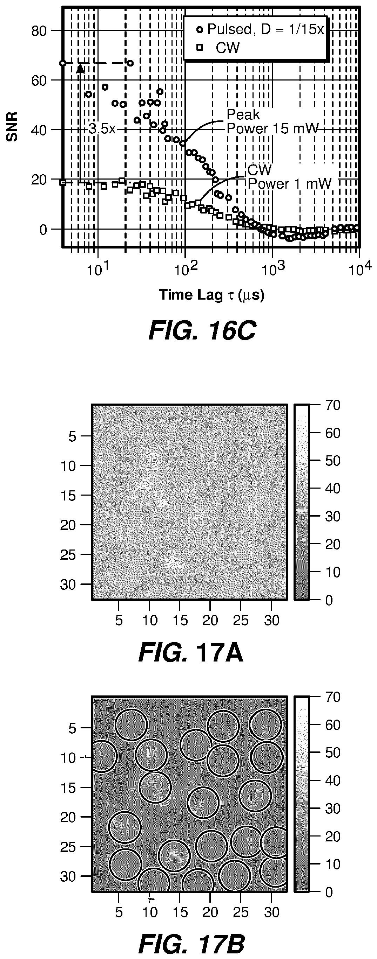

FIGS. 16A and 16B show that in order to keep the same average power below the MPE (28.5 mW at 785 nm), the laser input power may be increased by a factor of N (e.g., 15x) at a duty cycle of 1/N (e.g., 1/15). FIG. 16A shows that a 1 mW CW laser has the same average power with a 15 mW pulsed laser at 1/15 duty cycle. FIGS. 16B and 16C show results from an equilibrium homogeneous liquid phantom, showing a comparison of the resulting $g_2$ and SNR between the continuous wave and pulsed modes. FIG. 16B shows that $g_2$ remains similar. FIG. 16C shows that the SNR becomes 3.5x higher for the pulsed mode (this is in addition to the 32x SNR gain obtained from capturing 1024 speckles). Hence, in total SNR gain of greater than 100 may be achieved, which is effectively similar to the SNR expected from a 10,000-pixel SPAD array in CW mode.

FIGS. 17A - 17B show, by way of example, an image of a speckle pattern not using (FIG. 17A), and using (FIG. 17B) a speckle tracking method using object detection. The speckle size is about 5-7 pixels in diameter and the turnover time is about 1 ms. The number of speckles per frame varies between 15-25, with a mean of 21.

FIGS. 18A - 18J shows further example data obtained using various example approaches, including the formation of an example ensemble average autocorrelation function.

FIG. 18A illustrates formation of an example ensemble average autocorrelation function (which may also be referred to an ensemble mean correlation function, here labeled G2) as a function of time delay.

FIG. 18B and 18C illustrate variations of standard deviation (STD) and signal to noise ratio (SNR), respectively, for example ensemble average autocorrelation functions as a function of time delay.

FIG. 18D shows a variation in fit error for an example ensemble average autocorrelation function as a function of time. The fit error is generally small and shows little appreciable systemic error. There is a minor oscillatory component.

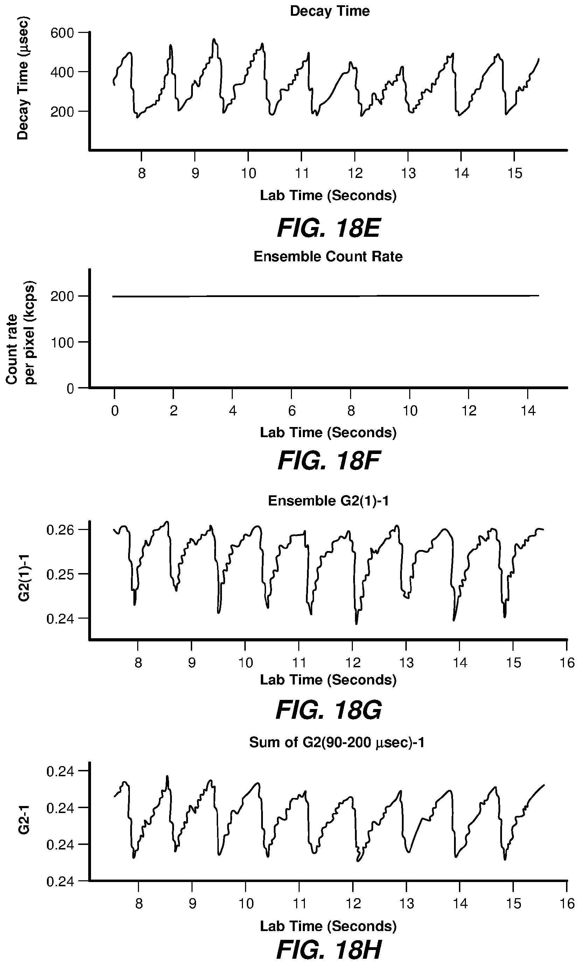

FIG. 18E shows variations in measured decay time as a function of time.

FIG. 18F illustrates that there is little appreciable variation in ensemble count rate as a function of time.

FIG. 18G shows variations in the value of $g_2(\tau = T_{bin}) - 1$ as a function of time.

FIG. 18H shows variations in the value of sum of $g_2(\tau) - 1$ in the range of $\tau = 90$ µs and $\tau = 200$ µs, as a function of time, for a subset of detected autocorrelation functions. The results are reasonably similar to those of FIG. 18E and 18G in this example measurement.

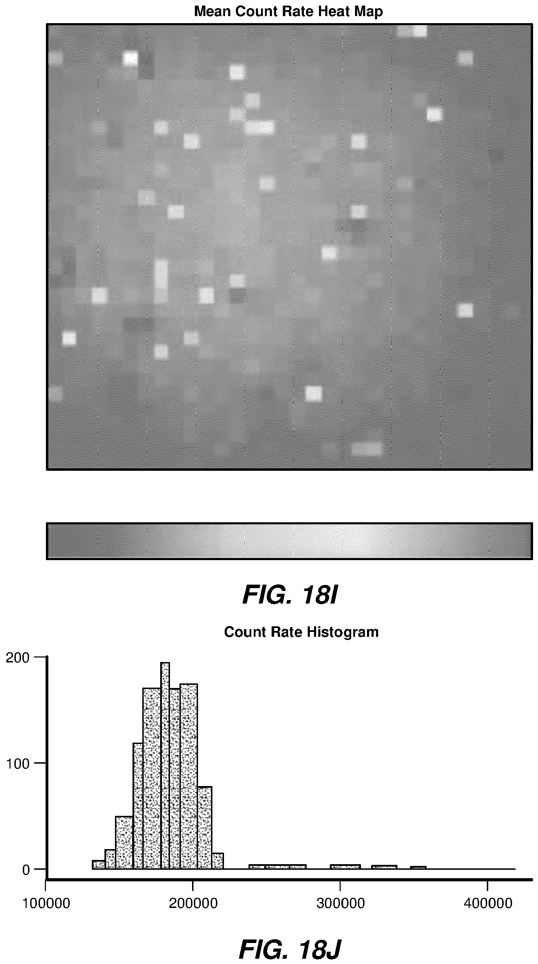

FIG. 18I shows a mean count rate heat map for an example detector array, showing the presence of hot pixels. In some examples, autocorrelation functions from hot pixels may be excluded from the ensemble average autocorrelation function.

FIG. 18J shows a count rate histogram for an example detector array. A relatively small number of individual detectors may show a relatively high dark count rate (e.g., corresponding to bars to the right of the histogram).

FIG. 19 is a flow diagram of an example method for fiber-based speckle contrast optical spectroscopy. An example method (1900) may include collecting scattered laser radiation using a detector fiber (1910); illuminating a detector array using the scattered laser radiation to form a plurality of speckles on the detector array (1920); and determining an ensemble average correlation function (1930), for example, based on the time-dependent intensity correlation function for each of a plurality of detectors of the detector array. An example method may further include Illuminating an object using laser radiation, such as laser radiation having a wavelength of between 700 nm and 900 nm. In some examples, the laser radiation may be pulsed laser radiation. The distance between an end of the detector fiber and the detector array may be adjusted so that a speckle area on the detector array is approximately equal to the detection area of a detector within the plurality of detectors. A detector area may be approximately determined by dividing the area of the detector array by the number of detectors within the detector array.

In some examples, a method may further include determining a characteristic time related to the object. The characteristic time of a dynamic physical process within the object may be determined based on the ensemble average autocorrelation function. The characteristic time may be related to fluid flow dynamics within the object, such as blood flow within a body part such as the head. In some examples, the controller may be configured to provide a controller output including a characteristic time determination (e.g., a characteristic of a dynamic process within the object) based on the ensemble average autocorrelation function.

FIG. 20 is a flow diagram of a further example method for fiber-based speckle contrast optical spectroscopy. The example method 2000 includes receiving scattered laser radiation from a body part of a person (2010), illuminating a detector array using the scattered laser radiation to form a plurality of speckles (2020), and determining an ensemble average correlation function (2030), for example, based on the time-dependent intensity autocorrelation function for each of a plurality of detectors of the detector array. An example method may further include illuminating the head of the person using laser radiation, such as visible and/or near-IR laser radiation. For example, laser radiation may include red and/or near-IR wavelengths. In some examples, a method may further include determining a characteristic time for a process within the body part, such as a characteristic time related to blood flow. In some examples, the body part may be an arm, wrist, hand, finger, leg, waist, or other body part of a person. In some examples, the body part may be the head of the user.

In some examples, a method may include receiving scattered radiation from an object, illuminating a detector array using the scattered radiation, and determining an ensemble average correlation function, for example, based on the time-dependent intensity correlation function for each of a plurality of detectors of the detector array. In some examples, the scattered radiation may be laser radiation. In some examples, the scattered radiation may be near-IR radiation, for example, having a wavelength between 700 nm and 900 nm. In some examples, the scattered radiation may be scattered laser radiation.

In some examples, a method may include placing a wearable device on a user, where the wearable device is configured to irradiate a body part of the user using laser radiation and collect scattered laser radiation from the body part. The wearable device may include a band (e.g., a strap encircling a body part), wristband, ring, belt, helmet, spectacles, or other wearable device. The controller may be part of the wearable device. In some examples, the controller may be in wireless or wired communication with the wearable device. In some examples, some or all functions of the controller may be provided by a remote computer, such as a remote server.

In some examples, a computer-implemented method may include performing one or more steps of an example method as described herein. For example, a computer-implemented method may include receiving data from a plurality of detectors, determining a time-dependent autocorrelation function for each of the plurality of detectors, and determining an ensemble average autocorrelation function based on the time-dependent autocorrelation function for each of the plurality of detectors. An example method may further include control of a laser; for example, energizing the laser to produce pulsed laser radiation. An example method may further include adjusting a distance between a fiber end from which laser radiation emerges and a detector array, for example, so that a speckle dimension may at least approximately match a detector area dimension.

In some examples, an apparatus may include a laser, a source fiber configured to receive laser radiation from the laser at one end and deliver the laser radiation to an object from the other end, a detector fiber receiving scattered laser radiation at one end and illuminating a detector array with the scattered laser radiation from the other end, forming speckles on the light receiving surface of the detector array. The detector array may include a plurality of detectors positioned to receive the scattered laser radiation from the end of the detector fiber. The distance between the detector array and the end of the detector fiber may be adjustable. A controller may be configured to receive detector data from the detector array, and, for each detector of the plurality of detectors, determine a time-dependent intensity autocorrelation function. The controller may be further configured to determine an ensemble average autocorrelation function based on the time-dependent intensity autocorrelation functions determined for each detector.

In some examples, an apparatus may include a band (e.g., at least one strap or other support structure) configured to attach the apparatus to the body part of the user. In some examples, the support structure may be a component of a wearable device, such as a wrist-band, helmet, visor, spectacles, hat, other head-mounted device, glove, ring, or other wearable device. In some examples, a method may further include wearing the wearable device so that a body part is illuminated by laser radiation (e.g., from one or more source fibers), and scattered laser radiation is collected by one or more detector fibers. In some examples, the object may be directed illuminated by a laser. For example, a laser may be directed at an object, such as a body part, and the source fiber may be omitted. In some examples, the laser radiation may be collimated (e.g., using one or more lasers or collimators). For example, a head-mounted device may support one or more lasers, and one or more detector fibers configured to detect scattered laser radiation. In some examples, a device may be worn on other body parts, for example, as a smart watch or wristband, on a forearm or other limb, or worn on a hand or finger (e.g., as a glove or ring).

In some examples, additional optical elements (such as lenses, filters, polarizers, splitters, or combiners) may be located between the detector end of the detector fiber and the detector array. For example, the position of one or more optical elements (such as one or more lenses) may be adjusted to adjust the speckle diameter on the detector array.

In some examples, an apparatus may use an interferometric mDCS approach. In some examples, a fraction of the incident laser radiation (e.g., 10% of the laser source emission) is redirected using a splitter, bypasses the diffuse object, and is combined with the diffused light (also termed scattered light) at the end of the detector fiber or combined directly with the speckle field at the detector array. This approach may be used to obtain stronger speckle contrast using an interferometric approach to increase the overall SNR of the mDCS.

In some examples, one or more additional optical elements (such as lenses, filters, polarizers, splitters, or combiners) may be located between the detector end of the detector fiber and the detector array.

In some examples, an apparatus may be configured as an interferometric mDCS, where unscattered laser radiation is directed (e.g., using a splitter) to bypass the object and is combined with the scattered light, for example, at an end of the detector fiber or combined directly with the speckle fields at the detector array. This approach may improve speckle contrast and may increase the overall SNR of the mDCS apparatus.

In some examples, a laser may have a coherence length of at least 1 m, for example, at least 5 m, and in some examples at least 9 m. In some examples, the coherence length of the laser radiation may be longer than the source-detector distance, which may be approximately 1 cm for some applications. However, a longer coherence length may give a higher amplitude autocorrelation function, which may give a higher SNR. The SNR may be improved by using a laser having a higher coherence length, such as at least 1 m.

In some examples, a laser may be a pulsed laser or a continuous-wave (CW) laser. For some examples, the pulsed laser may provide an improved SNR. However, in some examples, the CW laser may be less expensive and may be commercially advantageous in some applications. Also, in some examples, a CW laser and associate circuitry may be readily miniaturized, so a CW laser may be used in applications with a smaller form factor.

In some examples, the laser wavelength may be in the range 700 nm - 1200 nm, for example, in the range 700 nm - 900 nm. For example, a laser having an emission wavelength of 1064 nm (or greater) may be used provided the detectors have sufficient sensitivity at the appropriate wavelength. In some examples, detectors may include superconducting nanowire single photon detectors (SNSPD). Any suitable laser wavelength may be used, depending on the object under study. If the object is a human head, a wavelength in the range 700 nm - 1200 nm may be used. These example wavelengths may pass through a layer of hair, skin, and/or bone (e.g., the skull) and be scattered within the brain, allowing internal processes within the brain to be characterized. Similar wavelengths may be used for the characterization of other body parts.

In some examples, a source fiber may include at least one single-mode fiber, and a detector fiber may include at least one multi-mode fiber. In some examples, a source fiber may include at least one multi-mode fiber. In some examples, the detector fiber may include a plurality of fibers, such as a bundle of multimode fibers. In some examples, the detector fiber may include a plurality of multimode fibers to provide speckles for large-pixel-count array, a detector array having at least 1 million pixels. In some examples, the singular terms source fiber and/or detector fiber may be used for convenience to refer to fiber bundles. For example, a source fiber may include a multimode fiber, a bundle of single-mode fiber, or a bundle of multi-mode fiber, as long as the overall coherence length upon delivery is longer than the photon path length between the source and the detector.

In some examples, an apparatus may include a support configured to direct laser radiation at an object, and receive scattered laser radiation from the object. The support may be provided by a band, for example, a strap that may extend around a portion of the object. For example, the object may include a body part, and the band may encircle the body part. In some examples, one or more source fibers may be configured to illuminate the object, and one or more detector fibers may be configured to receive scattered laser radiation. The apparatus may further include a distance adjuster configured to adjust the distance between the detector end of the detector fiber and the detector array. Scattered laser radiation may be collected by the collector end of the detector fiber and may emerge from the detector end of the fiber to form a plurality of speckles on the detector array.

In some examples, the apparatus may include a controller configured to provide an output including a characteristic time based on the ensemble average autocorrelation function. The characteristic time may convey information about dynamic properties within the object, such as fluid flow dynamics, including blood flow dynamics. The controller may be configured to provide a time determination (such as a characteristic time) related to a dynamic process within an object illuminated by the laser radiation from the source fiber, such as fluid flow dynamics within the object, such as blood flow within a body part.

In some examples, the apparatus may include a wearable apparatus configured to be worn by a user. The apparatus may be configured so that laser radiation from the source fiber (or, in some examples, directly from a laser) may be directed into a body part of a user when the apparatus is worn by the user. The collector end of the detector fiber may receive scattered radiation from the body part of the user. A wearable device may include a head-mounted device, which may include a band (such as a strap having the appearance of a head band), helmet, visor, spectacles, or other head-mounted device.

In some examples, a two-dimensional or three-dimensional image relating to blood flow dynamics within brain (or other body part) of a user may be determined. An imaging apparatus may include one or more source fibers (and/or laser sources) and one or more detector fibers. The apparatus may have a plurality of different source - detector separations, and/or (in some examples) one or more source-detector separations may be varied during collection of image data. In some examples, one or more of the following may be adjusted during data collection: orientation of the apparatus (e.g., a band may at least partially rotate around a head of a user), source-detector separation, wavelength (or multiple wavelengths may be used), orientation of one or both of source and detector fibers, or other parameter.

In some examples, a system may include at least one physical processor, and physical memory including computer-executable instructions that, when executed by the physical processor, cause the physical processor to perform one or more steps of any example method described herein. In some examples, a non-transitory computer-readable medium may include one or more computer-executable instructions that, when executed by at least one processor of a computing device, cause the computing device to perform one or more steps of any example method described herein. An example method may include receiving data from a plurality of detectors, determining a time-dependent autocorrelation function for each of the plurality of detectors, and determining an ensemble average autocorrelation function based on the time-dependent autocorrelation function for each of the plurality of detectors.

In conclusion, an example apparatus may include a laser, a source fiber configured to deliver the laser radiation to an object (e.g., illuminating the object using laser radiation emerging from the delivery end of the source fiber), and a detector fiber configured to receive scattered laser radiation and illuminate a detector array with the scattered laser radiation to form speckles on the detector array. The detector fiber may have a collector end configured to receive scattered laser radiation from the object (e.g., laser radiation scattered by a dynamic process within the object) and a detector end configured to illuminate the detector array and form a plurality of speckles on the detector array. The laser may provide red and/or near-IR radiation when energized. The detector array may include a plurality of detectors, such as SPAD detectors or superconducting nanowire single photon (SNSPD) detectors, and may be positioned to receive scattered laser radiation from the detector end of the detector fiber. The distance between the detector array and the end of the detector fiber may be adjustable. A controller, which may include one or more processors, may be configured to receive detector data from the detector array, determine a time-dependent intensity autocorrelation function for each detector of a plurality of detectors, and determine an ensemble average autocorrelation function. The apparatus may provide information relating to dynamic processes within the object, such as blood flow in examples where the object is a body part of a user.

**Further Examples of the Invention**

Example 1: An apparatus may include a laser configured to provide laser radiation, a detector fiber having a collector end configured to receive scattered laser radiation and a detector end, a detector array including a plurality of detectors positioned to receive the scattered laser radiation from the detector end of the detector fiber, and a controller, configured to receive detector data for each detector of the plurality of detectors, determine a time-dependent intensity autocorrelation function for each detector of the plurality of detectors, and determine an ensemble average autocorrelation function based on the time-dependent intensity autocorrelation function for each detector of the plurality of detectors.

Example 2. The apparatus of example 1, further including a distance adjuster configured to adjust a distance between the detector end of the detector fiber and the detector array.

Example 3. The apparatus of any of examples 1 or 2, further including at least one optical element located between the detector end of the detector fiber and the detector array.

Example 4. The apparatus of any of examples 1 - 3, where the plurality of detectors includes an arrangement of single-photon avalanche diodes.

Example 5. The apparatus of any of examples 1 - 4, where the plurality of detectors includes at least 1000 detectors.

Example 6. The apparatus of any of examples 1 - 5, further including a source fiber, where the source fiber has a source end configured to receive the laser radiation from the laser and a delivery end, and the source fiber includes a single-mode fiber.

Example 7. The apparatus of any of examples 1 - 6, where the detector fiber includes a multimode fiber.

Example 8. The apparatus of any of examples 1 - 7, where the apparatus is configured so that the scattered laser radiation emerges from the detector end of the detector fiber to form a plurality of speckles on the detector array.

Example 9. The apparatus of any of examples 1 - 8, where the laser radiation has a wavelength of between 700 nm and 1200 nm.

Example 10. The apparatus of any of examples 1 - 9, where the laser radiation has a coherence length of at least 1 m.

Example 11. The apparatus of any of examples 1 -10, further including a beam-splitter configured to direct unscattered laser radiation to the detector array.

Example 12. The apparatus of any of examples 1 - 11, where the controller is configured to provide a controller output including time determination based on the ensemble average autocorrelation function.

Example 13. The apparatus of example 12, where the time determination is related to fluid flow dynamics within an object illuminated by the laser radiation.

Example 14. The apparatus of any of examples 1 - 13, where the apparatus is a wearable apparatus configured to be worn by a user, and the apparatus is configured so that the laser radiation is directed into a body part of the user when the apparatus is worn by the user, and the collector end of the detector fiber receives the scattered laser radiation from the body part of the user.

Example 15. The apparatus of any of examples 1 - 14, where the apparatus is a head-mounted device and the body part is a head of the user.

Example 16. The apparatus of any of examples 1 - 15, where the apparatus includes at least one band configured to attach the apparatus to the body part of the user.

Example 17. A method may include collecting scattered laser radiation using a detector fiber, illuminating a detector array using the scattered laser radiation to form a plurality of speckles on the detector array, and determining an ensemble average correlation function based on time-dependent intensity correlation functions for each of a plurality of detectors of the detector array.

Example 18. The method of example 17, further including adjusting a distance or a lens position between an end of the detector fiber and the detector array so that a speckle size on the detector array is approximately equal to a detector area within the plurality of detectors.

Example 19. The method of any of examples 17 or 18, where the scattered laser radiation has a wavelength of between 700 nm and 1200 nm.

Example 20. The method of any of examples 17 - 19, further including Illuminating an object using laser radiation, and determining a characteristic time related to fluid flow within the object from the ensemble average correlation function.

The invention may include or be implemented in conjunction with various types of artificial reality systems. Artificial reality is a form of reality that has been adjusted in some manner before presentation to a user, which may include, for example, a virtual reality, an augmented reality, a mixed reality, a hybrid reality, or some combination and/or derivative thereof. Artificial-reality content may include completely computer-generated content or computer-generated content combined with captured (e.g., real-world) content. The artificial-reality content may include video, audio, haptic feedback, or some combination thereof, any of which may be presented in a single channel or in multiple channels (such as stereo video that produces a three-dimensional (3D) effect to the viewer). Additionally, artificial reality may also be associated with applications, products, accessories, services, or some combination thereof, that are used to, for example, create content in an artificial reality and/or are otherwise used in (e.g., to perform activities in) an artificial reality.

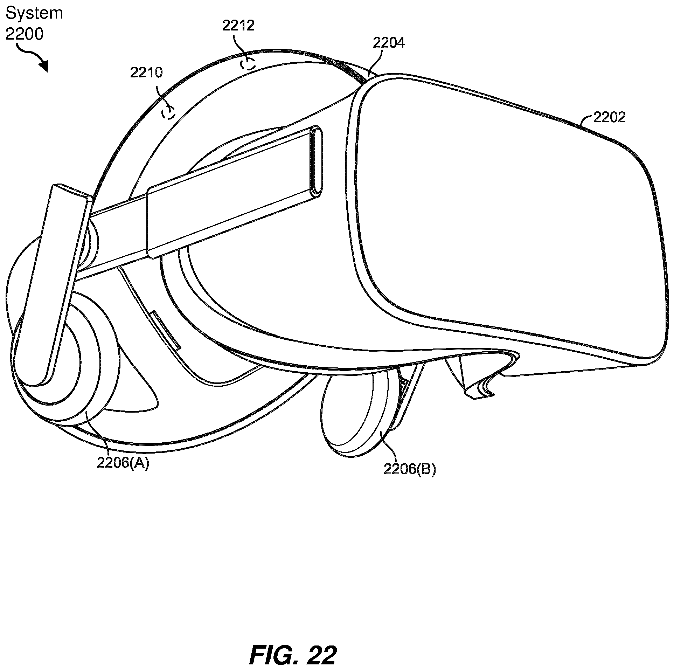

Artificial-reality systems may be implemented in a variety of different form factors and configurations. Some artificial reality systems may be designed to work without near-eye displays (NEDs). Other artificial reality systems may include an NED that also provides visibility into the real world (such as, e.g., an augmented-reality system 2100 as shown in FIG. 21) or that visually immerses a user in an artificial reality (such as, e.g., a virtual-reality system 2200 as shown in FIG. 22). While some artificial-reality devices may be self-contained systems, other artificial-reality devices may communicate and/or coordinate with external devices to provide an artificial-reality experience to a user. Examples of such external devices include handheld controllers, mobile devices, desktop computers, devices worn by a user, devices worn by one or more other users, and/or any other suitable external system.

Turning to FIG. 21, augmented-reality system 2100 may include an eyewear device 2102 with a frame 2110 configured to hold a left display device 2115(A) and a right display device 2115(B) in front of a user's eyes. Display devices 2115(A) and 2115(B) may act together or independently to present an image or series of images to a user. While augmented-reality system 2100 includes two displays, embodiments of this disclosure may be implemented in augmented-reality systems with a single NED or more than two NEDs.

The augmented-reality system 2100 may include one or more sensors, such as sensor 2140. Sensor 2140 may generate measurement signals in response to motion of augmented-reality system 2100 and may be located on substantially any portion of frame 2110. Sensor 2140 may represent one or more of a variety of different sensing mechanisms, such as a position sensor, an inertial measurement unit (IMU), a depth camera assembly, a structured light emitter and/or detector, or any combination thereof. The augmented-reality system 2100 may or may not include sensor 2140 or may include more than one sensor. In embodiments in which sensor 2140 includes an IMU, the IMU may generate calibration data based on measurement signals from sensor 2140. Examples of sensor 2140 may include, without limitation, accelerometers, gyroscopes, magnetometers, other suitable types of sensors that detect motion, sensors used for error correction of the IMU, or some combination thereof.

In some examples, the augmented-reality system 2100 may also include a microphone array with a plurality of acoustic transducers 2120(A)-2120(J), referred to collectively as acoustic transducers 2120. Acoustic transducers 2120 may represent transducers that detect air pressure variations induced by sound waves. Each acoustic transducer 2120 may be configured to detect sound and convert the detected sound into an electronic format (e.g., an analog or digital format). The microphone array in FIG. 21 may include, for example, ten acoustic transducers: 2120(A) and 2120(B), which may be designed to be placed inside a corresponding ear of the user, acoustic transducers 2120(C), 2120(D), 2120(E), 2120(F), 2120(G), and 2120(H), which may be positioned at various locations on frame 2110, and/or acoustic transducers 2120(1) and 2120(J), which may be positioned on a corresponding neckband 2105.

One or more of the acoustic transducers 2120(A)-(J) may be used as output transducers (e.g., speakers). For example, the acoustic transducers 2120(A) and/or 2120(B) may be earbuds or any other suitable type of headphone or speaker.

The configuration of acoustic transducers 2120 of the microphone array may vary. While augmented-reality system 2100 is shown in FIG. 21 as having ten acoustic transducers 2120, the number of acoustic transducers 2120 may be greater or less than ten. In some embodiments, using higher numbers of acoustic transducers 2120 may increase the amount of audio information collected and/or the sensitivity and accuracy of the audio information. In contrast, using a lower number of acoustic transducers 2120 may decrease the computing power required by an associated controller 2150 to process the collected audio information. In addition, the position of each acoustic transducer 2120 of the microphone array may vary. For example, the position of an acoustic transducer 2120 may include a defined position on the user, a defined coordinate on frame 2110, an orientation associated with each acoustic transducer 2120, or some combination thereof.

Acoustic transducers 2120(A) and 2120(B) may be positioned on different parts of the user's ear, such as behind the pinna, behind the tragus, and/or within the auricle or fossa. Or, there may be additional acoustic transducers 2120 on or surrounding the ear in addition to acoustic transducers 2120 inside the ear canal. Having an acoustic transducer 2120 positioned next to an ear canal of a user may enable the microphone array to collect information on how sounds arrive at the ear canal. By positioning at least two of acoustic transducers 2120 on either side of a user's head (e.g., as binaural microphones), augmented-reality device 2100 may simulate binaural hearing and capture a 3D stereo sound field around about a user's head. The acoustic transducers 2120(A) and 2120(B) may be connected to augmented-reality system 2100 via a wired connection 2130, or the acoustic transducers 2120(A) and 2120(B) may be connected to augmented-reality system 2100 via a wireless connection (e.g., a BLUETOOTH connection). Alternatively, acoustic transducers 2120(A) and 2120(B) may not be used at all in conjunction with augmented-reality system 2100.

Acoustic transducers 2120 on frame 2110 may be positioned in a variety of different ways, including along the length of the temples, across the bridge, above or below display devices 2115(A) and 2115(B), or some combination thereof. Acoustic transducers 2120 may also be oriented such that the microphone array is able to detect sounds in a wide range of directions surrounding the user wearing the augmented-reality system 2100. An optimization process may be performed during manufacturing of augmented-reality system 2100 to determine relative positioning of each acoustic transducer 2120 in the microphone array.

In some examples, an augmented reality device may be configured to support one or more source fibers and one or more detector fibers. For example, in relation to FIG. 21, the delivery end of a source fiber and/or the collector end of a detector fiber may be located, for example, at one or more of the locations indicated as possible locations for an audio transducer. In relation to FIG. 21, the delivery end of a source fiber may be located at or proximate locations such as 2120(A), 2120(B), 2120(C), 2120(D), and/or any other suitable location, and configured to direct laser radiation towards the head of the user when the user wears the head-mounted device. Acoustic transducers may also be located at these locations, as described above. The collector end of a detector fiber may be located at one of more of these example locations, and may be configured to collect scattered light from the head of the user when the user wears the head-mounted device.

A laser and/or detector array may be located with the frame of a head-mounted device, or within a module that may be attached to the head-mounted device.

In some examples, augmented-reality system 2100 may include or be connected to an external device (e.g., a paired device), such as neckband 2105. Neckband 2105 generally represents any type or form of paired device. Thus, the discussion of neckband 2105 may also apply to various other paired devices, such as charging cases, smart watches, smart phones, wrist bands, hat, ring, other wearable devices, hand-held controllers, tablet computers, laptop computers, other external compute devices, etc.

In some examples, the paired device may include an mDCS apparatus, and may include, for example, a light source, detector fiber, detector array, and other components such as those described herein. A paired device may include a neck-band, watch, smart phone, wrist band, chest band, hat, ring, other jewelry item or bodily adornment, other wearable device, smart shoe, clothing item, hand-held controller, tablet computer, laptop computer, other external computer devices, etc. A chest band may include an apparatus configured to monitor cardiac function, for example, including one or more of an mDCS apparatus, pulse oximeter, electrocardiograph, or other component. A device component, such as a band (e.g., a strap or other support component), may encircle a limb or other body part, and monitor, for example, blood flow, blood velocity, and/or other circulatory parameter.

As shown, neckband 2105 may be coupled to eyewear device 2102 via one or more connectors. The connectors may be wired or wireless and may include electrical and/or non-electrical (e.g., structural) components. In some cases, eyewear device 2102 and neckband 2105 may operate independently without any wired or wireless connection between them. While FIG. 21 illustrates the components of eyewear device 2102 and neckband 2105 in example locations on eyewear device 2102 and neckband 2105, the components may be located elsewhere and/or distributed differently on eyewear device 2102 and/or neckband 2105. The components of eyewear device 2102 and neckband 2105 may be located on one or more additional peripheral devices paired with eyewear device 2102, neckband 2105, or some combination thereof.

Pairing external devices, such as neckband 2105, with augmented-reality eyewear devices may enable the eyewear devices to achieve the form factor of a pair of glasses while still providing sufficient battery and computation power for expanded capabilities. Some or all of the battery power, computational resources, and/or additional features of augmented-reality system 2100 may be provided by a paired device or shared between a paired device and an eyewear device, thus reducing the weight, heat profile, and form factor of the eyewear device overall while still retaining desired functionality. For example, neckband 2105 may allow components that would otherwise be included on an eyewear device to be included in neckband 2105 since users may tolerate a heavier weight load on their shoulders than they would tolerate on their heads. Neckband 2105 may also have a larger surface area over which to diffuse and disperse heat to the ambient environment. Thus, neckband 2105 may allow for greater battery and computation capacity than might otherwise have been possible on a stand-alone eyewear device. Since weight carried in neckband 2105 may be less invasive to a user than weight carried in eyewear device 2102, a user may tolerate wearing a lighter eyewear device and carrying or wearing the paired device for greater lengths of time than a user would tolerate wearing a heavy standalone eyewear device, thereby enabling users to more fully incorporate artificial reality environments into their day-to-day activities.

Neckband 2105 may be communicatively coupled with eyewear device 2102 and/or to other devices. These other devices may provide certain functions (e.g., tracking, localizing, depth mapping, processing, storage, etc.) to augmented-reality system 2100. In the embodiment of FIG. 21, neckband 2105 may include two acoustic transducers (e.g., 2120(1) and 2120(J)) that are part of the microphone array (or potentially form their own microphone subarray). Neckband 2105 may also include a controller 2125 and a power source 2135.

Acoustic transducers 2120(1) and 2120(J) of neckband 2105 may be configured to detect sound and convert the detected sound into an electronic format (analog or digital). In the embodiment of FIG. 21, acoustic transducers 2120(1) and 2120(J) may be positioned on neckband 2105, thereby increasing the distance between the neckband acoustic transducers 2120(1) and 2120(J) and other acoustic transducers 2120 positioned on eyewear device 2102. In some cases, increasing the distance between acoustic transducers 2120 of the microphone array may improve the accuracy of beamforming performed via the microphone array. For example, if a sound is detected by acoustic transducers 2120(C) and 2120(D) and the distance between acoustic transducers 2120(C) and 2120(D) is greater than, for example, the distance between acoustic transducers 2120(D) and 2120(E), the determined source location of the detected sound may be more accurate than if the sound had been detected by acoustic transducers 2120(D) and 2120(E).

Controller 2125 of neckband 2105 may process information generated by the sensors on neckband 2105 and/or augmented-reality system 2100. For example, controller 2125 may process information from the microphone array that describes sounds detected by the microphone array. For each detected sound, controller 2125 may perform a direction-of-arrival (DOA) estimation to estimate a direction from which the detected sound arrived at the microphone array. As the microphone array detects sounds, controller 2125 may populate an audio data set with the information. In embodiments in which augmented-reality system 2100 includes an inertial measurement unit, controller 2125 may compute all inertial and spatial calculations from the IMU located on eyewear device 2102. A connector may convey information between augmented-reality system 2100 and neckband 2105 and between augmented-reality system 2100 and controller 2125. The information may be in the form of optical data, electrical data, wireless data, or any other transmittable data form. Moving the processing of information generated by augmented-reality system 2100 to neckband 2105 may reduce weight and heat in eyewear device 2102, making it more comfortable to the user.

Power source 2135 in neckband 2105 may provide power to eyewear device 2102 and/or to neckband 2105. Power source 2135 may include, without limitation, lithium ion batteries, lithium-polymer batteries, primary lithium batteries, alkaline batteries, or any other form of power storage. In some cases, power source 2135 may be a wired power source. Including power source 2135 on neckband 2105 instead of on eyewear device 2102 may help better distribute the weight and heat generated by power source 2135.

As noted, some artificial reality systems may, instead of blending an artificial reality with actual reality, substantially replace one or more of a user's sensory perceptions of the real world with a virtual experience. One example of this type of system is a head-worn display system, such as virtual-reality system 2200 in FIG. 22, that mostly or completely covers a user's field of view. Virtual-reality system 2200 may include a front rigid body 2202 and a band 2204 shaped to fit around a user's head. Virtual-reality system 2200 may also include output audio transducers 2206(A) and 2206(B). Furthermore, while not shown in FIG. 22, front rigid body 2202 may include one or more electronic elements, including one or more electronic displays, one or more inertial measurement units (IMUs), one or more tracking emitters or detectors, and/or any other suitable device or system for creating an artificial-reality experience.

In some examples, the band 2204 (or other portion of a head-mounted device) may be configured to support one or more source fibers and one or more detector fibers. For example, in relation to FIG. 22, the delivery end of a source fiber may be located, for example, at 2210 and configured to direct laser radiation towards the head of the user when the user wears the head-mounted device. The collector end of a detector fiber may be located at, for example, at 2212 and configured to collect scattered light from the head of the user when the user wears the head-mounted device. The detector fiber may direct the collected light (e.g., scattered laser radiation) to the detector array which may be located within the housing of the head-mounted device. There may be one or more other and/or alternative locations for source fibers and/or detector fibers. The head-mounted device may also support or enclose (e.g., within a housing) one or more laser, one or more detector arrays, and any suitable optical and/or electrooptical components. A head-mounted device may also include a controller that may be configured to provide the described controller functions of an mDCS system as well as AR/VR related functions.

Artificial reality systems may include a variety of types of visual feedback mechanisms. For example, display devices in augmented-reality system 2100 and/or virtual-reality system 2200 may include one or more liquid crystal displays (LCDs), light emitting diode (LED) displays, microLED displays, organic LED (OLED) displays, digital light project (DLP) micro-displays, liquid crystal on silicon (LCoS) micro-displays, and/or any other suitable type of display screen. These artificial reality systems may include a single display screen for both eyes or may provide a display screen for each eye, which may allow for additional flexibility for varifocal adjustments or for correcting a user's refractive error. Some of these artificial reality systems may also include optical subsystems having one or more lenses (e.g., conventional concave or convex lenses, Fresnel lenses, adjustable liquid lenses, etc.) through which a user may view a display screen. These optical subsystems may serve a variety of purposes, including to collimate (e.g., make an object appear at a greater distance than its physical distance), to magnify (e.g., make an object appear larger than its actual size), and/or to relay (to, e.g., the viewer's eyes) light. These optical subsystems may be used in a non-pupil-forming architecture (such as a single lens configuration that directly collimates light but results in so-called pincushion distortion) and/or a pupil-forming architecture (such as a multilens configuration that produces so-called barrel distortion to nullify pincushion distortion).

In addition to or instead of using display screens, some of the artificial reality systems described herein may include one or more projection systems. For example, display devices in augmented-reality system 2100 and/or virtual-reality system 2200 may include micro-LED projectors that project light (using, e.g., a waveguide) into display devices, such as clear combiner lenses that allow ambient light to pass through. The display devices may refract the projected light toward a user's pupil and may enable a user to simultaneously view both artificial reality content and the real world. The display devices may accomplish this using any of a variety of different optical components, including waveguide components (e.g., holographic, planar, diffractive, polarized, and/or reflective waveguide elements), light-manipulation surfaces and elements (such as diffractive, reflective, and refractive elements and gratings), coupling elements, etc. Artificial reality systems may also be configured with any other suitable type or form of image projection system, such as retinal projectors used in virtual retina displays.

In some examples, an augmented reality or virtual reality system may include head-mounted device configured to detect scattered laser radiation from the head of a user, as described in detail herein. One or more source fibers may be arranged around the exterior of a person's head, and one or more detector fibers may be arranged to detect scattered laser radiation. In some examples, a near-eye device may be used as a support structure for one or more source fibers and/or one or more detector fibers.

The artificial reality systems described herein may also include various types of computer vision components and subsystems. For example, augmented-reality system 2100 and/or virtual-reality system 2200 may include one or more optical sensors, such as two-dimensional (2D) or 3D cameras, structured light transmitters and detectors, time-of-flight depth sensors, single-beam or sweeping laser rangefinders, 3D LiDAR sensors, and/or any other suitable type or form of optical sensor. An artificial reality system may process data from one or more of these sensors to identify a location of a user, to map the real world, to provide a user with context about real-world surroundings, and/or to perform a variety of other functions.

The artificial reality systems described herein may also include one or more input and/or output audio transducers. Output audio transducers may include voice coil speakers, ribbon speakers, electrostatic speakers, piezoelectric speakers, bone conduction transducers, cartilage conduction transducers, tragus-vibration transducers, and/or any other suitable type or form of audio transducer. Similarly, input audio transducers may include condenser microphones, dynamic microphones, ribbon microphones, and/or any other type or form of input transducer. In some embodiments, a single transducer may be used for both audio input and audio output.

The artificial reality systems described herein may also include tactile (i.e., haptic) feedback systems, which may be incorporated into headwear, gloves, body suits, handheld controllers, environmental devices (e.g., chairs, floormats, etc.), and/or any other type of device or system. Haptic feedback systems may provide various types of cutaneous feedback, including vibration, force, traction, texture, and/or temperature. Haptic feedback systems may also provide various types of kinesthetic feedback, such as motion and compliance. Haptic feedback may be implemented using motors, piezoelectric actuators, fluidic systems, and/or a variety of other types of feedback mechanisms. Haptic feedback systems may be implemented independent of other artificial reality devices, within other artificial reality devices, and/or in conjunction with other artificial reality devices.

By providing haptic sensations, audible content, and/or visual content, artificial reality systems may create an entire virtual experience or enhance a user's real-world experience in a variety of contexts and environments. For instance, artificial reality systems may assist or extend a user's perception, memory, or cognition within a particular environment. Some systems may enhance a user's interactions with other people in the real world or may enable more immersive interactions with other people in a virtual world. Artificial reality systems may also be used for educational purposes (e.g., for teaching or training in schools, hospitals, government organizations, military organizations, business enterprises, etc.), entertainment purposes (e.g., for playing video games, listening to music, watching video content, etc.), and/or for accessibility purposes (e.g., as hearing aids, visual aids, etc.). The embodiments disclosed herein may enable or enhance a user's artificial reality experience in one or more of these contexts and environments and/or in other contexts and environments.

As detailed above, the computing devices and systems described and/or illustrated herein broadly represent any type or form of computing device or system capable of executing computer-readable instructions, such as those contained within the modules described herein. In their most basic configuration, these computing device(s) may each include at least one memory device and at least one physical processor.

In some examples, the term "memory device" generally refers to any type or form of volatile or non-volatile storage device or medium capable of storing data and/or computer-readable instructions. In one example, a memory device may store, load, and/or maintain one or more of the modules described herein. Examples of memory devices include, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In some examples, the term "physical processor" generally refers to any type or form of hardware-implemented processing unit capable of interpreting and/or executing computer-readable instructions. In one example, a physical processor may access and/or modify one or more modules stored in the above-described memory device. Examples of physical processors include, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the modules described and/or illustrated herein may represent portions of a single module or application. In addition, one or more of these modules may represent one or more software applications or programs that, when executed by a computing device, may cause the computing device to perform one or more tasks. For example, one or more of the modules described and/or illustrated herein may represent modules stored and configured to run on one or more of the computing devices or systems described and/or illustrated herein. One or more of these modules may also represent all or portions of one or more special-purpose computers configured to perform one or more tasks.

In addition, one or more of the modules described herein may transform data, physical devices, and/or representations of physical devices from one form to another. For example, one or more of the modules recited herein may receive light scattering data (such as detector data) to be transformed, transform the detector data (e.g., by forming an ensemble average autocorrelation function), output a result of the transformation (e.g., a characteristic time of a dynamic process), use the result of the transformation to provide a characterization of the dynamic process, and store the result of the transformation (e.g., to determine the time dependence of a dynamic process, such as a fluid flow process such as blood flow). Additionally or alternatively, one or more of the modules recited herein may transform a processor, volatile memory, non-volatile memory, and/or any other portion of a physical computing device from one form to another by executing on the computing device, storing data on the computing device, and/or otherwise interacting with the computing device.

The term "computer-readable medium" generally refers to any form of device, carrier, or medium capable of storing or carrying computer-readable instructions. Examples of computer-readable media include, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed. The various exemplary methods described and/or illustrated herein may also omit one or more of the steps described or illustrated herein or include additional steps in addition to those disclosed.

The preceding description has been provided to enable others skilled in the art to best utilize various aspects of the exemplary embodiments disclosed herein. This exemplary description is not intended to be exhaustive or to be limited to any precise form disclosed. Many modifications and variations are possible without departing from the spirit and scope of the present disclosure. The embodiments disclosed herein should be considered in all respects illustrative and not restrictive. Reference may be made to the appended claims and their equivalents in determining the scope of the present disclosure.

Unless otherwise noted, the terms "connected to" and "coupled to" (and their derivatives), as used in the specification and claims, are to be construed as permitting both direct and indirect (i.e., via other elements or components) connection. In addition, the terms "a" or "an," as used in the specification and claims, are to be construed as meaning "at least one of." Finally, for ease of use, the terms "including" and "having" (and their derivatives), as used in the specification and claims, are interchangeable with and have the same meaning as the word "comprising."

## Claims

**1.** An apparatus comprising:

a laser, configured to provide laser radiation;
a detector fiber, having a collector end configured to receive scattered laser radiation and a detector end;
a detector array comprising a plurality of detectors positioned to receive the scattered laser radiation from the detector end of the detector fiber; and
a controller, configured to:

receive detector data for each detector of the plurality of detectors;
determine a time-dependent intensity autocorrelation function for each detector of the plurality of detectors; and
determine an ensemble average autocorrelation function based on the time-dependent intensity autocorrelation function for each detector of the plurality of detectors.

**2.** The apparatus of claim 1, further comprising a distance adjuster configured to adjust a distance between the detector end of the detector fiber and the detector array.

**3.** The apparatus of claim 1, further comprising at least one optical element located between the detector end of the detector fiber and the detector array.

**4.** The apparatus of claim 1, wherein the plurality of detectors includes an arrangement of single-photon avalanche diodes; and/or
wherein the plurality of detectors comprises at least 1000 detectors.

**5.** The apparatus of claim 1 further comprising a source fiber, wherein:

the source fiber has a source end configured to receive the laser radiation from the laser and a delivery end; and the source fiber includes a single-mode fiber; and/or
wherein the detector fiber includes a multimode fiber.

**6.** The apparatus of claim 1, wherein the apparatus is configured so that the scattered laser radiation emerges from the detector end of the detector fiber to form a plurality of speckles on the detector array.

**7.** The apparatus of claim 1, wherein the laser radiation has a wavelength of between 700 nm and 1200 nm; and/or wherein the laser radiation has a coherence length of at least 1 m.

**8.** The apparatus of claim 1, further comprising a beam-splitter configured to direct unscattered laser radiation to the detector array.

**9.** The apparatus of claim 1, wherein the controller is configured to provide a controller output including time determination based on the ensemble average autocorrelation function.

**10.** The apparatus of claim 9, wherein the time determination is related to fluid flow dynamics within an object illuminated by the laser radiation.

**11.** The apparatus of claim 1, wherein the apparatus is a wearable apparatus configured to be worn by a user, and the apparatus is configured so that:

the laser radiation is directed into a body part of the user when the apparatus is worn by the user; and
the collector end of the detector fiber receives the scattered laser radiation from the body part of the user.

**12.** The apparatus of claim 11, wherein the apparatus is a head-mounted device and the body part is a head of the user; and/or
wherein the apparatus includes at least one band configured to attach the apparatus to the body part of the user.

**13.** A method, comprising:

collecting scattered laser radiation using a detector fiber;
illuminating a detector array using the scattered laser radiation to form a plurality of speckles on the detector array; and
determining an ensemble average correlation function based on time-dependent intensity correlation functions for each of a plurality of detectors of the detector array.

**14.** The method of claim 13, further comprising adjusting a distance or a lens position between an end of the detector fiber and the detector array so that a speckle size on the detector array is approximately equal to a detector area within the plurality of detectors; and/or
wherein the scattered laser radiation has a wavelength of between 700 nm and 1200 nm.

**15.** The method of claim 13, further including:

Illuminating an object using laser radiation; and
determining a characteristic time related to fluid flow within the object from the ensemble average correlation function.

Camera 142

Camera 144

Camera 146

Camera 148

Camera 149

Source Light 110

100 102 122 124 126 128 129 132 134 136 138 139 150 152 154

FIG. 1

200
214
SMF
218
204
216
MMF
208
ρ
206
222
z
CW Laser
785 nm
32 x 32
SPAD Array
Object
202
210
212
220
Controller
230

FIG. 2A

242
244
248
SMF
Laser
Radiation
To SPAD Array
MMF
246
Rotating Diffuser
Phantom
240

FIG. 2B

Z = 106 mm
d
59 mm
30 mm
Decreasing z
0
10
20
30
40
Photon Counts
Photon Counts
80
60
40
20
0
Decorrelation
Time $\tau_C$
Time (ms)
$g_2(\tau)$ -1 (norm)
1
0.5
0
$\tau_C$
Time Lag $\tau$ ($\mu$S)

FIG. 2C

FIG. 2D

FIG. 2E

300
310
Wearable Device
316(1)-(n)
Cameras
312
Source Light
Optical Fibers
314(1)-(n)
User
302
320
321
Processor
Speckle Fields
322(1)-(n)
324(1)-(n)
Correlation Time
336
Input
334
Activity-to-input Map
332
Brain Activity
330
Blood Movement
340
Computing System

FIG. 3

Projection
414
Camera (Detector Array)
412
Distance
Adjuster
426
430
428
Lens Distance
Adjuster
420
424
Optical
Fiber
402
422

*FIG. 4*

1 Pixel
4 STD
g2(τ) -1
Time Lag τ (μS)
1024 Pixel
4 STD
g2(τ) -1
Time Lag τ (μS)
MDCS Noise Model
Pixel
1024
512
256
64
16
1
SNR
Time Lag τ (μS)
Experiment
SNR Gain = √M
SNR Gain
Number of Pixels, M

FIG. 5A

FIG. 5B

FIG. 5C

FIG. 5D

880 Pixels
32 Pixels
1 Pixel
Increasing M
~100 Counts
~4 Counts
D
E
SNR (4μS)
Count Rate Per Pixel (cps)
1024 Pixels
32 Pixels
1 Pixel
32x M
32x T_int
A
B
C
SNR (4μS)
Integration Time (ms)

FIG. 6B

FIG. 6A

5
10
15
20
25
30
5
10
15
20
25
30
0
10
20
30
40
50
60
70

*FIG. 7A*

Number of Speckles
Frame
0
100
200
300
400
500
0
5
10
15
20
25
30
35

*FIG. 7B*

Number of Speckles
Counts
0
100
150
0
5
10
15
20
25
30
35

*FIG. 7C*

Z = 59 mm
106 mm
132 mm
Photon Counts
Photon Counts
Photon Counts
Photon Counts
Photon Counts
Photon Counts

FIG. 8A

FIG. 8B

FIG. 8C

FIG. 8D

FIG. 8E

FIG. 8F

Fiber Core Diameter
200 µm
400 µm
600 µm
Diameter (Pixel)
Fiber-SPAD Distance (mm)
0
5
10
15
0
50
100
150

FIG. 8H

z= 106 mm
d
Data
Guess
2D Autocorrelation
Pixel
0
0.2
0.4
0.6
0.8
1
-15
-10
-5
0
5
10
15

FIG. 8G

2x2 px
4x4
16x16
Cluster size
1x1 Px
3x3 Px
5x5 Px
10x10 Px
32x32 Px
2D g2(τ)-1
Time Lag t (ms)
$g_2(\tau) = 1 + \beta\,|g_1(\tau)|^2$
β
Cluster Length (Pixel)

FIG. 9A

FIG. 9B

FIG. 9C

FIG. 9D

FIG. 9E

FIG. 9F

0.8
0.6
0.4
0.2
0
β
10
20
30
Cluster length (Pixel)
Speckle's Diameter
1.2 px
2.4px
3.6 px
4.8px
6.1 px
7.2 px
8.5 px
9.6 px
1
0.8
0.6
0.4
0.2
0
β(normalized)
2
4
6
8
10
12
14
Cluster Length (Pixel)
12
10
8
6
4
2
0
Cluster length at β-half (Pixel)
Cluster Length
Speckle's Diameter
50
100
150
200
Fiber-SPAD Distance (mm)

FIG. 10A

FIG. 10B

FIG. 10C

Cluster
R=3
R=2
R=1
1100
1102
Time

*FIG. 11A*

Ensemble
1110

*FIG. 11B*

1 Cluster (5x5 px)
0.5
0.4
0.3
0.2
001
0
-0.1
g2(τ)-1
4 STD
10^1
10^2
10^3
10^4
Time Lag τ (μs)

*FIG. 11C*

Ensemble of 36 Clusters
0.5
0.4
0.3
0.2
01
0
-0.1
g2(τ)-1
4 STD
10^1
10^2
10^3
10^4
Time Lag τ (μs)

*FIG. 11D*

35
30
25
20
15
10
5
0
SNR
$10^1$
$10^2$
$10^3$
$10^4$
Time Lag τ (μs)

*FIG. 11E*

35
30
25
20
15
10
5
0
SNR
6X SNR
$10^1$
$10^2$
$10^3$
$10^4$
Time Lag τ (μs)

*FIG. 11F*

Fiber Core Diameter
200 µm
600 µm
400 µm
910 µm
350
300
250
200
150
100
50
0
SNR (4µs)
5
10
15
20
25
30
Fiber- SPAD Distance (mm)

*FIG. 12A*

Fiber Core Diameter
200 µm
600 µm
400 µm
910 µm
350
300
250
200
150
100
50
0
SNR (4µs)
0.01
0.1
1
10
Number of Speckles (per 7x7µm2)

*FIG. 12B*

Number of Pixels
1000
800
600
400
200
0
883
141
1024
Cold
Hot
All
DCR Mean (kcps)
25
20
15
10
5
0
21
24233
3354
Cold
Hot
All


FIG. 13A

FIG. 13B

FIG. 13C

Cold Pixels (883)
All Pixels (1024)
Hot Pixels (141)
0.25
0.2
0.15
0.1
0.05
0
g2(τ)-1
10^1
10^2
10^3
10^4
Time Lag τ (μs)


*FIG. 13D*

Cold Pixels (883)
All Pixels (1024)
Hot Pixels (141)
70
60
50
40
30
20
10
0
SNR
10^1
10^2
10^3
10^4
Time Lag τ (μS)


*FIG. 13E*

Number of Pixels
250
200
150
100
50
0
Cool
Pixels(878)
Hot Pixels (146)
$10^0$
$10^1$
$10^2$
$10^3$
$10^4$
$10^5$
$10^6$
Dark Count Rate, DCR(cps)


*FIG. 14A*


Cool Pixels
All Pixels
Hot Pixels
$g_2$-1(4μs)
0.4
0.3
0.2
0.1
0
$10^3$
$10^4$
$10^5$
Count Rate per Pixel, $N_{ph}$ (cps)


*FIG. 14B*


Cool Pixels
All Pixels
Hot Pixels
SNR(4μs)
$10^3$
$10^2$
$10^1$
$10^0$
$10^{-1}$
~3.8E3
~2.6E4
$10^3$
$10^4$
$10^5$
Count Rate per Pixel, $N_{ph}$ (cps)


*FIG. 14C*

Rotating Diffuser Plate
$e^{-\tau^2/\tau_c^2}$
$e^{-\tau/\tau_c}$
1
0.8
0.6
0.4
0.2
0
$g_2(\tau)$-1 (a.u.)
$10^1$
$10^2$
$10^3$
$10^4$
Time Lag $\tau$ ($\mu$s)

*FIG. 15A*

Liquid Phantom
$e^{-\tau^2/\tau_c^2}$
$e^{-\tau/\tau_c}$
0.2
0.15
0.1
0.05
0
$g_2(\tau)$-1
$10^1$
$10^2$
$10^3$
$10^4$
Time Lag $\tau$ ($\mu$s)

*FIG. 15B*

Tint = 100ms
Tint*D = 6.7ms
D = Duty Cycle(1/15x)
Peak Power
CW
Input Power
15
10
5
0
0
0.1
0.2
0.3
0.4
0.5
0.6
0.7
0.8
0.9
1
Measurement Time (Sec)

*FIG. 16A*

Pulsed, D = 1/15x
CW
0.25
0.2
0.15
0.1
0.05
0
g2(τ)-1
10^1
10^2
10^3
10^4
Time Lag τ (μs)

*FIG. 16B*

Pulsed, D = 1/15x
CW
SNR
80
60
40
20
0
3.5x
Peak
Power 15 mW
CW
Power 1 mW
$10^1$
$10^2$
$10^3$
$10^4$
Time Lag τ (μs)

*FIG. 16C*

70
60
50
40
30
20
10
0
5
10
15
20
25
30

*FIG.* **17A**

*FIG. 17B*

G2 Ensemble Mean
G2
1.3
1.2
1.1
1.0
0.0001
0.001
0.01
Delay (seconds)

*FIG. 18A*

STD of G2 Ensemble Mean
STD
0.04
0.02
0
0.0001
0.001
0.01
Delay (seconds)

*FIG. 18B*

SNR of G2 Ensemble Mean
SNR
20
10
0
0.0001
0.001
0.01
Delay (seconds)

*FIG. 18C*

Fit Error
Fit Error
0.006
0.004
0.002
0
Lab Time (Seconds)

*FIG. 18D*

Decay Time
Decay Time (μsec)
600
400
200
8
9
10
11
12
13
14
15
Lab Time (Seconds)

*FIG. 18E*

Ensemble Count Rate
Count rate per pixel (kcps)
200
100
0
0
2
4
6
8
10
12
14
Lab Time (Seconds)

*FIG. 18F*

Ensemble G2(1)-1
G2(1)-1
0.26
0.25
0.24
8
9
10
11
12
13
14
15
16
Lab Time (Seconds)

*FIG. 18G*

Sum of G2(90-200 μsec)-1
G2-1
0.24
0.24
0.24
0.24
8
9
10
11
12
13
14
15
16
Lab Time (Seconds)

*FIG. 18H*

Mean Count Rate Heat Map

*FIG. 18I*

Count Rate Histogram
200
100
0
100000
200000
300000
400000

*FIG. 18J*

Method
1900
Start
Collect scattered laser radiation using a detector fiber
1910
Illuminate a detector array using the scattered laser radiation to form a plurality of speckles on the detector array
1920
Determine an ensemble average correlation function based on a plurality of detector autocorrelation functions
1930
End

*FIG. 19*

Method
2000
Start
Receive scattered laser radiation from a body part of a person
2010
Illuminate a detector array using the scattered laser radiation to form a plurality of speckles
2020
Determine an ensemble average correlation function based on the time-dependent intensity correlation function for each of a plurality of detectors of the detector array
2030
End

*FIG. 20*

System
2100
2110
2120(D)
2120(E)
2120(C)
2150
2120(G)
2120(F)
2120(B)
2102
2120(H)
2120(A)
2115(A)
2140
2130
2115(B)
2120(I)
2135
2120(J)
2125
2105

FIG. 21

System
2200
2212
2204
2210
2202
2206(A)
2206(B)

*FIG. 22*

Europäisches Patentamt
European Patent Office
Office européen des brevets

# EUROPEAN SEARCH REPORT

Application Number
EP 21 17 1362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DIETSCHE G ET AL: "Fiber-based multispeckle detection for time-resolved di ffusing-wave spectroscopy: characterization and applica tion to blood flow detection in deep tissue", APPLIED OPTICS, OPTICAL SOCIETY OF AMERICA, WASHINGTON, DC, US, vol. 46, no. 35, 10 December 2007 (2007-12-10), pages 8506-8514, XP001510503, ISSN: 0003-6935, DOI: 10.1364/AO.46.008506 | 1,3,4,6, 7,9-15 | INV. A61B5/026 A61B5/00 |
| Y | * page 8506, left-hand column, paragraph 1 - page 8508, right-hand column, paragraph 2 * * page 8511, left-hand column, last paragraph - right-hand column, paragraph 1 * ----- | 5,8 | |
| X | JUN LI, MARKUS NINCK, THOMAS GISLER: "Changes in scalp and cortical blood flow during hyperventilation measured with diffusing-wave spectroscopy", PROC. OF SPIE-OSA BIOMEDICAL OPTICS, vol. 7368, 7 July 2009 (2009-07-07), pages 1-8, XP040498134, * page 2, paragraph 4 - page 3, paragraph 2 * ----- | 1,4,6,7, 9-15 | TECHNICAL FIELDS SEARCHED (IPC) A61B |
| Y | WO 2019/204231 A1 (UNIV CALIFORNIA [US]) 24 October 2019 (2019-10-24) * paragraph [0004] * * paragraph [0008] * * paragraph [0037] * * paragraph [0039] * ----- -/-- | 5,8 | |

The present search report has been drawn up for all claims

1

| Place of search | Date of completion of the search | Examiner |
|---|---|---|
| Berlin | 1 September 2021 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

# EUROPEAN SEARCH REPORT

Application Number
EP 21 17 1362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | LIU WENHUI ET AL: "Fast and sensitive diffuse correlation spectroscopy with highly parallelized single photon detection", APL PHOTONICS, AMERICAN INSTITUTE OF PHYSICS, 2 HUNTINGTON QUADRANGLE, MELVILLE, NY 11747, vol. 6, no. 2, 12 February 2021 (2021-02-12), pages 1-14, XP012253855, DOI: 10.1063/5.0031225 [retrieved on 2021-02-12] * page 2, right-hand column, paragraph 2 - page 3, column 3, paragraph 1 * * page 4, right-hand column, paragraph 3 - page 5, column 3, paragraph 1 * * figures 1,2 * ----- | 1-15 | |

TECHNICAL FIELDS SEARCHED (IPC)

The present search report has been drawn up for all claims

| Place of search | Date of completion of the search | Examiner |
|---|---|---|
| Berlin | 1 September 2021 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 17 1362

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2021

| Patent document cited in search report | Publication date | Patent family member(s) | Publication date |
|---|---|---|---|
| WO 2019204231 A1 | 24-10-2019 | US 2021030284 A1 | 04-02-2021 |
| | | WO 2019204231 A1 | 24-10-2019 |

EPO FORM P0459

For more details about this annex : see Official Journal of the European Patent Office, No. 12/82